# FASCICULE DE BREVET EUROPEEN

(11) **EP 2 326 655 B1**
(45) Date de publication et mention de la délivrance du brevet: **31.12.2014**
(21) Numéro de dépôt: 09780902.4
(22) Date de dépôt: 22.07.2009
(51) Int. Cl.: C07F 9/30, C07F 9/32, A61K 31/662, A61K 31/675, A61K 31/485, A61K 31/352, A61K 31/197, A61P 25/04

(54) **DERIVES AMINOPHOSPHINIQUES UTILES DANS LE TRAITEMENT DE LA DOULEUR**
AMINOPHOSPHINSÄUREDERIVATE, DIE BEI DER BEHANDLUNG VON SCHMERZEN VERWENDUNG FINDEN KÖNNEN
AMINOPHOSPHINIC DERIVATIVES THAT CAN BE USED IN THE TREATMENT OF PAIN

(30) Priorité: 23.07.2008 FR 0855015
(43) Date de publication de la demande: 01.06.2011
(60) Demande divisionnaire: 14157306.3
(73) Titulaire: Pharmaleads, 75013 Paris (FR)
(72) Inventeur: ROQUES, Bernard, F-75014 Paris (FR); PORAS, Hervé, F-78870 Bailly (FR); FOURNIE-ZALUSKI, Marie-Claude, F-75011 Paris (FR)
(74) Mandataire: Regimbeau
(86) Numéro de dépôt international: PCT/EP2009/059394
(87) Numéro de publication internationale: WO 2010/010106

(56) Documents cités:
- WO-A-97/00261
- US-A- 5 476 847
- CHEN H ET AL: "Long lasting antinociceptive properties of enkephalin degrading enzyme (NEP and APN) inhibitor prodrugs." JOURNAL OF MEDICINAL CHEMISTRY 11 OCT 2001, vol. 44, no. 21, 11 octobre 2001 (2001-10-11), pages 3523-3530, XP002514724 ISSN: 0022-2623 cité dans la demande
- ZHOU JIA ET AL: "NAAG peptidase inhibitors and their potential for diagnosis and therapy." NATURE REVIEWS. DRUG DISCOVERY DEC 2005, vol. 4, no. 12, décembre 2005 (2005-12), pages 1015-1026, XP002514725 ISSN: 1474-1776

## Description

La présente invention concerne des composés aminophosphiniques, leur procédé de préparation et leur utilisation dans le traitement de la douleur telle que les douleurs neuropathiques, neuroinflammatoires, post-opératoires ou les douleurs vives par excès de nociception.

La perception, la transmission et la régulation des influx nociceptifs sont sous la dépendance de plusieurs neurotransmetteurs, et en particulier des enképhalines. Les enképhalines (Met-enképhaline et Leu-enképhaline) sont des pentapeptides initialement trouvés dans le cerveau de mammifères (Hugues Nature 1975, 258, 577). Les enképhalines se lient principalement à deux classes de récepteurs, les récepteurs µ et δ (Lord et al. Nature 1977, 267,495) dont les fonctions et les localisations sont différentes (Waksman et al. Proc. Natl. Acad. Sci. 1986, 83,152).

Les propriétés antinociceptives des enképhalines ont été démontrées après administration par voie intracérébroventriculaire d'enképhalines exogènes (Belluzi Nature 1976, 260, 625). Cependant cette réponse est très fugace à cause d'une métabolisation très rapide de ces peptides par des activités enzymatiques. Des analogues d'enképhalines synthétiques, modifiés pour les rendre résistants à la dégradation enzymatique ont montré des propriétés antinociceptives égales à celles de la morphine, mais ont également présenté les mêmes effets secondaires indésirables que la morphine.

D'autre part, on sait que les enképhalines (Tyr-Gly-Gly-Phe-Met et Tyr-Gly-Gly-Phe-Leu) sont physiologiquement inactivées par deux métallopeptidases à zinc, la néprilysine (EC 3.4.24.11, NEP) qui clive la liaison Gly³-Phe⁴ (Malfroy et al. Nature 1978, 276, 523) et l'aminopeptidase N (EC 3.4.11.2, APN) qui coupe la liaison Tyr¹-Gly² de ces peptides (Waksman et al. Eur. J. Pharmacol. 1985, 117, 233; Roques et al. Pharmacological Reviews 1993, 45, 87-146).

L'inhibition de ces deux activités enzymatiques, en protégeant complètement les enképhalines endogènes de leur dégradation enzymatique (Bourgoin et al. J. Pharm. Exp. Ther. 1986, 238, 360), révèle les activités pharmacologiques et en particulier analgésiques et antidépressives de ces neuropeptides (Roques Trends Pharmacol. Sci. 2000, 21, 475 ; Jutkiewicz CNS Drugs Reviews 2007, 13, 192-206).

Des travaux récents ont démontré que le système enképhalinergique, constitué des enképhalines, des enzymes d'inactivation NEP et APN et des récepteurs opioïdes, était présent au niveau des nocicepteurs, c'est à dire sur les terminaisons extrêmement fines des nerfs sensitifs qui transmettent les influx douloureux (M.J. Millan Prog. in Neurobiology, 57, 1999,1-164). Ainsi :
i) le gène de la préproenképhaline est exprimé dans les ganglions dorsaux des nerfs spinaux, puis transporté à la périphérie au niveau des nocicepteurs (Antunes-Bras J et al. Neuroscience 2001, 103, 1073-1083),
ii) les enképhalines sont exprimées en grande quantité dans les cellules immunitaires attirées vers les tissus lésés (Przewlocki R et al. Neuroscience 1992, 48(2), 491-500) et libérées de ces cellules sur le site de la lésion (Rittner HL et al. FASEB J. 2006, 20, 2627-2629),
iii) les récepteurs opioïdes sont présents sur les terminaisons périphériques (Hassan AHS et al. Neuroscience 1993, 55, 185-195),
iv) enfin l'activité des deux peptidases NEP et APN est augmentée au niveau des leucocytes recrutés par l'inflammation (Salzet M et al. Trends in Neuroscience 2000, 23, 550-555).

Les inhibiteurs mixtes des deux activités enzymatiques, décrits dans l'art antérieur sont des pro-drogues que l'on peut classer en deux grandes familles.

La première famille correspond à des dérivés d'aminoacides qui associent, par un pont disulfure, un puissant inhibiteur de NEP et un puissant inhibiteur d'APN (FR 2 651229, J. Med. Chem. 1992, 35, 2473). Ces molécules présentent une excellente activité antinociceptive par voie intraveineuse (*iv*). Une nouvelle génération de molécules plus solubles a permis d'obtenir des composés présentant une bonne activité par voie orale (FR 2 892 413, FR 2 892 414 et FR 08/53092).

La deuxième famille comprend des composés qui inhibent conjointement l'APN et la NEP. Ce sont soit des composés à fonction hydroxamate (FR 2 518 088 et FR 2 605 004), soit des composés aminophosphiniques (FR 2 755 135 et FR 2 777 780).

Les composés à fonction hydroxamate, décrits dans ces documents, présentent une excellente activité *in vitro* et *in vivo* après administration par voie intracérébroventriculaire. Ceci a été particulièrement démontré dans les publications suivantes (Eur. J. Pharmacol. 1984, 102, 525-528 ; Eur. J. Pharmacol. 1989, 165, 199-207 ; Eur. J. Pharmacol. 1991, 192, 253-262). Une activité significative a également pu être observée après administration *iv* dans un modèle de rat arthritique (Brain Research 1989,497,94-101).

Les composés aminophosphiniques décrits dans les demandes FR 2 755 135 et FR 2 777 780 présentent au niveau de l'atome d'azote terminal soit une fonction amine libre soit une fonction imine. Or, il a été constaté par les inventeurs que, dans des conditions physiologiques, les composés ayant une telle fonction imine n'inhibent pas l'activité des deux peptidases NEP et APN. Les inventeurs ont découvert que pour assurer l'activité il est important que la pro-drogue régénère une fonction amine libre, ce qui n'est pas possible en présence d'une imine, dans des conditions physiologiques.

Dans les composés décrits dans la demande FR 2 755 135, la fonction acide phosphinique est laissée libre ou est protégée par un groupement protecteur qui est un alkyle ou un benzyle. Mais il a ensuite été décrit que l'activité diminue lorsque la fonction acide phosphinique n'est pas protégée (Hecker S. J. et Erion M. D. J. Med. Chem. 2008, 51, 2328-2345).

Dans les composés décrits dans la demande FR 2 777 780, la fonction acide phosphinique est protégée par un groupement protecteur qui est :
- soit un groupe -CH(X)-O-C(O)-Y, avec X, Y= alkyle ou phényle ;
- soit un groupement ester de S-acylthioéthyle (SATE) de formule -CH₂-CH₂-S-CO-W, avec W = alkyle ou phényle.

Toutefois, le groupement SATE ne peut pas être utilisé en thérapie humaine du fait de la toxicité du produit cyclique (sulfure d'éthylène) généré par l'hydrolyse du thioester dans le corps humain (Hecker S. J. et Erion M. D. J. Med. Chem. 2008, 51, 2328-2345).

Les inventeurs ont en outre constaté, pour la première fois, que la présence conjointe d'un groupe -CH(X)-O-C(O)-Y, protecteur de la fonction acide phosphinique, et d'une fonction amine libre conduit à la formation d'un produit de transfert inactif (formation d'un amide -N-C(O)-Y qui n'est pas hydrolysable ; voir exemple 13).

Cependant, dans le cas des dérivés aminophosphiniques décrits dans la demande FR 2 777 780, une bonne activité antinociceptive avec une longue durée d'action a été démontrée sur des modèles animaux de nociception après administration par voie *iv* ou *ip* (intrapéritonéale) lorsque les molécules étudiées ont été solubilisées dans un mélange d'huile, d'éthanol et d'eau (J. Med. Chem. 2000, 43, 1398-1408 ; J. Med. Chem. 2001, 44, 3523-3530 ; Pain 2003, 104, 139-148). Toutefois, aucune de ces molécules n'a été suffisamment soluble dans un soluté administrable chez l'homme, et aucune activité antinociceptive significative n'a été détectée après administration par voie orale.

Les documents US 5 476 847, WO 97/00263 et Nature rev. 2005, 4, 1015-26 décrivent des dérivés d'acide phosphonique répondant à la formule générale (I) pour laquelle R1 ne représente pas un groupement -C(=O)-O-C(R⁸)(R⁹)-OC(=O)-R¹⁰, utiles pour le traitement de maladies cardiovasculaires (US 5 476 847, WO 97/00263) ou pour le traitement de douleurs neuropathiques et inflammatoires (Nature rev. 2005, 4, 1015-26).

L'un des objets de l'invention est donc de fournir de nouveaux composés de type aminophosphinique stables, capables d'inhiber conjointement et avec une longue durée d'action (c'est-à-dire au moins 2h) les deux activités enzymatiques (néprilysine et aminopeptidase N) responsables de la dégradation des enképhalines et donc d'amplifier de manière importante les propriétés pharmacologiques de ces peptides après administration orale ou après mise en solution dans un soluté compatible avec une administration chez l'homme.

Dans ce but, la fonction amine primaire des inhibiteurs aminophosphiniques a été substituée par des groupements de protection temporaire physiologiquement acceptables, la fonction acide carboxylique a été estérifiée ou non et la fonction acide phosphinique est laissée libre ou est substituée par des groupements de protection temporaire physiologiquement acceptables. Ces protections ont conduit à des molécules très stables présentant une très bonne biodisponibilité.

Un autre objet de l'invention est de fournir de nouveaux composés qui présentent les propriétés des substances morphiniques, en particulier un puissant effet analgésique sur les différents types de douleur (aiguë, inflammatoire, neuropathique, etc.), des effets bénéfiques sur le comportement (diminution de la composante émotionnelle de la douleur), ainsi que des effets périphériques (antidiarréique, antitussique, etc.) sans en avoir les inconvénients majeurs (tolérance, dépendances physique et psychique, dépression respiratoire, constipation, nausée, vomissements, sédation, etc.).

Un autre objet de l'invention est de proposer des associations entre les composés de la présente invention et des composés connus pour leurs propriétés antinociceptives mais présentant à fortes doses des effets secondaires néfastes. Ces associations concernent plus particulièrement la morphine et ses dérivés, le THC (tétrahydrocannabinol) et ses dérivés ainsi que les dérivés du Gaba tels que la Gabapentine ou la Prégabaline. On a en effet pu constater une forte potentialisation des réponses antinociceptives obtenues par combinaison de doses subactives d'un des composés revendiqués dans la présente demande et d'un des analgésiques précédemment cités (morphine, THC, Gabapentine). De même les composés selon l'invention peuvent être avantageusement associés, dans le cadre du traitement de douleurs neuropathiques, à une des toxines botuliques, injectée localement (Ranoux D. et al., 2008, Anal. Neurol., 64, 274-283). Les composés de l'invention peuvent également être associés à des antagonistes des récepteurs purinergiques, en particulier du récepteur P2X3, dont l'un des antagoniste sélectif est le composé A-317491 (Wu et al., 2004, Eur J. Pharm., 504, 45-53)

L'invention a ainsi plus particulièrement pour objet des composés répondant à la formule générale (I) suivante :

R₁-NH-CH(R₂)-P(=O)(OR₃)-CH₂-C(R₄)(R₅)-CONH-CH(R₆)-COOR₇ (I)

ou un sel pharmaceutiquement acceptable de celui-ci,
pour laquelle:
▪ R₁ représente un groupement -C(=O)-O-C(R⁸)(R⁹)-OC(=O)-R¹⁰, dans lequel
   o R⁸ et R⁹ représentent, indépendamment l'un de l'autre, un atome d'hydrogène ou un groupe alkyle,
   o et R¹⁰ représente un groupe alkyle,
▪ R₂ représente:
   - un groupe alkyle linéaire ou ramifié, comportant de 1 à 6 atomes de carbone,
   - un groupe phényle;
▪ R₃ représente un atome d'hydrogène ou un groupe de formule -C(R¹²)(R¹³)-OC(=O)-R¹⁴, dans lequel
   o R¹² et R¹³ représentent, indépendamment l'un de l'autre, un atome d'hydrogène ou un groupe alkyle,
   o et R¹⁴ représente un groupe alkyle,
▪ R₅ représente un atome d'hydrogène et R₄ représente :
   - un benzyle éventuellement substitué sur le noyau phényle par :
      ∘ 1 à 5 atome(s) d'halogène tel(s) que le fluor ou le brome ;
      o un groupe OR¹⁵ ou SR¹⁵, dans lequel R¹⁵ représente un atome d'hydrogène, un groupe benzyle ou une chaîne hydrocarbonée, saturée ou insaturée, linéaire ou ramifiée, comportant de 1 à 4 atomes de carbone ;
      o un groupe amino ;
      o un groupe CF₃ ;
      o un groupe phényle ; ou
      o un cycle hétéroaromatique à 5 ou 6 chaînons ;
      R₄ et R₅ forment ensemble, avec le carbone qui les porte :
   - un cycle hydrocarboné saturé à 5 ou 6 chaînons ; ou
   - un cycle pipéridine, l'azote se trouvant en position 4 et étant éventuellement substitué par :
      * un groupe -SO₂-Ph ;
      * un groupe CF₃ ;
      * un groupe alkyle en C₁ à C₄ ;
      * un groupe acyle en C₁ à C₄ ;
      * un phényle ou un benzyle éventuellement substitué par un ou plusieurs atome(s) d'halogène, un groupe alkyle en C₁ à C₄ ou un groupe alcoxy en C₁ à C₄; ou
      * un hétérocycle aromatique tel qu'une pyridine ou une pyrimidine, éventuellement substitué par un groupe alkyle en C₁ à C₄ ou un groupe alcoxy en C₁ à C₄ ;
▪ R₆ représente :
   - un groupe alkyle linéaire ou ramifié, comportant de 1 à 6 atomes de carbone, et éventuellement substituée par :
      o un groupe OR¹⁶, dans lequel R¹⁶ représente un atome d'hydrogène,
▪ R₇ représente un radical choisi dans le groupe constitué par un atome d'hydrogène, un benzyle, un alkyle en C₂ à C₄, -CHR¹⁸-COOR¹⁹, -CHR¹⁸-OC(=O)R¹⁹ et -CHR¹⁸-OC(=O)OR¹⁹, dans lesquels R¹⁸ et R¹⁹ représentent, indépendamment l'un de l'autre, un groupe alkyle, aryle, arylalkyle, cycloalkyle, cyclohétéroalkyle, hétéroalkyle, hétéroaryle ou hétéroarylalkyle.

Dans la présente invention, on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Par « sels pharmaceutiquement acceptables » d'un composé, on entend désigner dans la présente invention des sels qui sont pharmaceutiquement acceptables, comme défini ici, et qui possèdent l'activité pharmacologique souhaitée du composé parent. Dans le cadre de la présente invention, il s'agit de sels obtenus avec une base minérale ou organique. Ainsi, le sel formé correspond :
- soit au remplacement d'un proton acide par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium,
- soit à la coordination de ce proton acide avec une base organique ou inorganique.

Les bases organiques acceptables comprennent des amines telles que l'ammoniaque, la diéthanolamine, l'éthanolamine, N-méthylglucamine, la triéthanolamine, la triéthylamine, la trométhamine et similaires. Les bases inorganiques acceptables comprennent l'hydroxyde d'aluminium, l'hydroxyde de calcium, l'hydroxyde de lithium (lithine), l'hydroxyde de potassium (potasse), le carbonate de sodium et l'hydroxyde de sodium (soude).

Avantageusement, les sels pharmaceutiquement acceptables des composés de l'invention seront des sels obtenus avec une base minérale ou organique pharmaceutiquement acceptable, telle que la lithine, la soude, la potasse, l'ammoniaque, une amine tertiaire de formule NRₐR_{b}R_{c}, où Rₐ, R_{b} et R_{c} représentent, indépendamment les uns de autres, un groupe alkyle tel que défini ci-dessous, comme la triéthylamine, ou encore un aminoacide basique tel que la lysine ou l'arginine et leurs dérivés.

Par « insaturé », on entend, au sens de la présente invention, que la chaîne hydrocarbonée comprend une ou plusieurs insaturation(s). Par « insaturation », on entend, au sens de la présente invention, une double ou une triple liaison.

Par « atome d'halogène », on entend, au sens de la présente invention, un atome de fluor, de chlore, de brome ou d'iode. Avantageusement, il s'agit d'un atome de fluor, de brome ou de chlore. Encore avantageusement, il s'agit d'un atome de fluor ou de brome, et de préférence de fluor.

Par groupe « amino », on entend, au sens de la présente invention, un groupe de formule -NR'R", où R' et R" représentent, indépendamment l'un de l'autre, un atome d'hydrogène ou un groupe hydrocarboné saturé ou insaturé, linéaire, ramifié ou cyclique, comportant de 1 à 6, de préférence de 1 à 4, atomes de carbone, R' et R" ne pouvant représenter en même temps un atome d'hydrogène, ou R' et R" forment ensemble, avec l'atome d'azote qui les porte, un hétérocycle à 5 ou 6 chaînons, saturé ou non, et ne comportant pas d'autre hétéroatome que l'azote qui porte les deux radicaux R' et R". En particulier, le groupe amino peut être un groupe -NHMe, -NHEt, -NHPr, -NHiPr, -NHBu, -NHiBu, -NHtBu, pipéridinyle ou pyrrolidinyle.

Par groupement « aryle », on entend, au sens de la présente invention, un groupement aromatique, comportant de préférence de 5 à 10 atomes de carbone, sauf mention contraire, et comprenant un ou plusieurs cycles accolés, comme par exemple un groupement phényle ou naphtyle. Avantageusement, il s'agit du phényle.

Par groupement « hétéroaryle », on entend, au sens de la présente invention, tout groupe aryle tel que défini ci-dessus dans lequel un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, un atome d'azote pouvant être éventuellement oxydé sous forme de N-oxyde. Des exemples de groupes hétéroaryle sont les groupes furyle, thiényle, pyrrolyle, pyridinyle, pyrimidyle, pyrazolyle, imidazolyle, tétrazolyle ou encore indyle.

Par « cycle hétéroaromatique à 5 ou 6 chaînons », on entend, au sens de la présente invention, un groupe hétéroaryle tel que défini ci-dessus ne comportant qu'un seul cycle à 5 ou 6 chaînons. Il s'agit notamment d'un groupe thiényle, pyrrolyle, pyridinyle, pyrimidyle, pyrazolyle, imidazolyle ou encore tétrazolyle.

Par « hétérocycle », on entend, au sens de la présente invention, un cycle hydrocarboné, avantageusement à 5 ou 6 chaînons, dont un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydés sous forme de N-oxyde et de S-oxyde. Sauf mention contraire, ce cycle pourra être saturé ou aromatique.

Dans le cas où le ou les hétéroatome(s) est (sont) choisi(s) parmi l'azote et le soufre, l'hétérocycle peut être en particulier un groupe : pipéridinyle, pyrrolidinyle, pyrrolyle, thiényle, pyrrazolyle, imidazolyle, pyridinyle, pyridazinyle, pyrimidinyle, pipérazinye, thiadiazolyle, tétrahydrothiényle ou encore thiazolyle.

Par « alkyle », on entend, au sens de la présente invention, une chaîne hydrocarbonée saturée, linéaire ou ramifiée, comportant de 1 à 6 atomes de carbone, sauf mention contraire. Il s'agit en particulier des groupes méthyle, éthyle, n-propyle, isopropyle, n-butyle, iso-butyle, sec-butyle, tert-butyle, n-pentyle, n-hexyle.

Par « hétéroalkyle », on entend, au sens de la présente invention, une chaîne hydrocarbonée saturée, linéaire ou ramifiée, comportant de 1 à 5 atomes de carbone et 1 ou 2 hétéroatomes, tels que par exemple des atomes de soufre, azote ou oxygène.

Par « acyle en C₁ à C₄ », on entend, au sens de la présente invention, un groupe alkyle tel que défini ci-dessus, comportant de 1 à 4 atomes de carbone, lié à la molécule par l'intermédiaire d'un groupement CO. Il peut s'agir en particulier d'un groupement acétyle, formyl et propionyl.

Par « cycloalkyle », on entend, au sens de la présente invention, un cycle hydrocarboné saturé comportant de 3 à 7, avantageusement de 5 à 7, atomes de carbone, en particulier le groupe cyclohexyle, cyclopentyle ou cycloheptyle.

Par « cyclohétéroalkyle », on entend, au sens de la présente invention, un groupe cycloalkyle tel que défini ci-dessus dans lequel un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydés sous forme de N-oxyde et de S-oxyde. Il peut s'agir en particulier d'un groupe pipéridinyle, pyrrolidinyle, tétrahydrofuryle, tétrahydrothénule, morpholinyle ou encore pipérazinyle.

Par « alcoxy », on entend, au sens de la présente invention, un groupe alkyle tel que défini ci-dessus lié à la molécule par l'intermédiaire d'un atome d'oxygène. Il s'agit en particulier d'un groupe méthoxy, éthoxy, n-propoxy, isopropoxy, n-butoxy, iso-butoxy, sec-butoxy ou encore tert-butoxy.

Par « arylalkyle », on entend, au sens de la présente invention, un groupe aryle tel que défini ci-dessus lié à la molécule par l'intermédiaire d'un groupe alkyle tel que défini ci-dessus. Il s'agit en particulier d'un groupe benzyle (Bn).

Par « hétéroarylalkyle », on entend, au sens de la présente invention, un groupe hétéroaryle tel que défini ci-dessus lié à la molécule par l'intermédiaire d'un groupe alkyle tel que défini ci-dessus. Il s'agit en particulier d'un groupe thénylméthyle ou furylméthyle.

De manière avantageuse, le radical R₁ représente un groupe -C(=O)-O-C(R⁸)(R⁹)-OC(=O)-R¹⁰ dans lequel :
- R⁸ et R⁹ représentent, indépendamment les uns des autres, un atome d'hydrogène et un groupe alkyle ; et
- R¹⁰ représente un groupe alkyle.

En particulier, le radical R₁ représente le groupe -(C=O)O-CHMe-OC(=O)CHMe₂.

De préférence, le radical R₂ représente un groupe alkyle, aryle ou arylalkyle ou hétéroarylalkyle, en particulier un groupe méthyle, phényle.

Selon un premier mode de réalisation, qui est également le mode de réalisation préféré, le radical R₃ représente un atome d'hydrogène.

En effet, les inventeurs on constate de manière surprenante que les composés de l'invention présentent une bonne activité même lorsque la fonction phosphinique reste libre, contrairement à l'enseignement de l'art antérieur (Hecker S. J. et Erion M. D. J. Med. Chem. 2008, 51, 2328-2345) qui indique que l'activité diminue lorsque la fonction phosphinique n'est pas protégée.

Selon un second mode de réalisation, le radical R₃ représente un groupe de formule -C(R¹²)(R¹³)-OC(=O)-R¹⁴, avec R¹², R¹³ et R¹⁴ tels que définis ci-dessus. En particulier, les radicaux R¹² et R¹³ représentent indépendamment l'un de l'autre un atome d'hydrogène ou un groupe alkyle et R¹⁴ représente un groupe alkyle. Il peut être avantageux que R¹² = R⁸, R¹³ = R⁹ et R¹⁴ = R¹⁰.

Ainsi, le radical R₃ représente avantageusement un atome d'hydrogène ou un groupe -CHMe-OC(=O)CHMe₂, et de préférence représente un atome d'hydrogène.

Selon une variante avantageuse de l'invention, R₅ représente un atome d'hydrogène et R₄ représente un groupe benzyle éventuellement substitué par 1 à 5 atome(s) d'halogène tel(s) que le fluor ou le brome, un phényle ou un cycle hétéroaromatique à 5 ou 6 chaînons. En particulier, R₅ représente un atome d'hydrogène et R₄ représente un groupe benzyle substitué, en position para, par un atome d'halogène, tel qu'un atome de brome, ou par un phényle.

Selon une autre variante avantageuse de l'invention, R₄ et R₅ forment ensemble, avec le carbone qui les porte, un cyclohexane ou un cycle pipéridine, l'azote se trouvant en position 4 et étant éventuellement substitué comme défini précédemment pour ces radicaux (en particulier, les substituants peuvent être un groupe -SO₂-Ph, un groupe acyle en C₁ à C₄, un groupe phényle).

De préférence, le radical R₆ représente un groupe alkyle tel qu'un groupe méthyle.

De manière également avantageuse, le radical R₇ représente un atome d'hydrogène ou un groupe alkyle, tel qu'un éthyle, ou un benzyle ou un groupe - CH(CH₃)-O-C(=O)-O-Et. De préférence, il s'agit d'un atome d'hydrogène ou d'un groupe benzyle.

Selon une variante avantageuse de l'invention, les radicaux ont la signification suivante :
- R₁ représente un groupe -C(=O)-O-C(R⁸)(R⁹)-OC(=O)-R¹⁰ dans lequel R⁸ représente un atome d'hydrogène et R⁹ et R¹⁰ représentent un groupe alkyle ;
- R₂ représente un groupe alkyle, phényle;
- R₃ représente un atome d'hydrogène ;
- R₅ représente un atome d'hydrogène et R₄ représente un groupe benzyle substitué en position para par un atome d'halogène (brome) ou par un phényle; ou R₄ et R₅ forment ensemble, avec le carbone qui les porte, un cyclohexane ou un cycle pipéridine, l'azote se trouvant en position 4 et étant éventuellement substitué par un groupe -SO₂-Ph, un groupe acyle en C₁ à C₄, ou un groupe phényle ;
- R₆ représente un groupe alkyle ;
- R₇ représente un atome d'hydrogène ou un groupe alkyle (tel qu'un éthyle) ou un benzyle ou un groupe -CH(CH₃)-O-C(=O)-O-Et.

Selon un mode de réalisation particulier, le composé de l'invention est choisi parmi les composés suivants :
Ester benzylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Ester éthoxycarbonyloxy de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3-{(1-isobutyryloxy-éthoxy)-[1-(1-isobutyryloxy-éthoxycarbonylamino)-éthyl]-phosphinoyl}-propionylamino)-propionique
Ester benzylique de l'acide 2-(2-(4-Bromo-benzyl)-3-{hydroxy-[1-(1-isobutyryloxy-éthoxycarbonylamino)-éthyl]-phosphinoyl}-propionylamino)-propionique
Acide 2-(2-(4-Bromo-benzyl)-3-{hydroxy-[1-(1-isobutyryloxyéthoxycarbonyl-amino)-éthyl]-phosphinoyl}-propionylamino)-propionique
Acide 2-[2-{Hydroxy-[(1-isobutyryloxy-éthoxycarbonylamino)-phényl-méthyl]-phosphinoylméthyl}-3-(4-thiophèn-3-yl-phényl)-propionylamino]-propionique
Acide 2-[2-{Hydroxy-[(1-isobutyryloxy-éthoxycarbonylamino)-phényl-méthyl]-phosphinoylméthyl}-3-(4-thiophèn-3-yl-phényl)-propionylamino]-3-hydroxypropionique
Acide 2-(3-Biphényl-4-yl-2-{hydroxy-[(1-isobutyryloxy-éthoxycarbonylamino)-thiophèn-3-yl-méthyl]-phosphinoylméthyl}-propionylamino)-propionique
Acide 2-{3-Biphényl-4-yl-2-[hydroxy-(1-isobutyryloxyméthoxy carbonylamino-éthyl)-phosphinoylméthyl]-propionylamino}-propionique
Acide 1-(1-{[3-biphényl-4-yl-2-(1-carboxy-éthylcarbamoyl)-propyl]-hydroxy-phosphinoyl}-éthylcarbamoyloxy)-éthyl de l'acide 2-diméthyl-propionique
Acide 2-[(1-{Hydroxy-[1-(1-isobutyryloxy-éthoxycarbonylamino)-éthyl]-phosphinoylméthyl}-cyclopentanecarbonyl)-amino]-propionique
Acide 2-[(1-Acétyl-4-{hydroxy-[1-(1-isobutyryloxy-éthoxy carbonylamino)-éthyl]-phosphinoylméthyl}-piperidine-4-carbonyl)-amino]-propionique
Acide 2-[(4-{Hydroxy-[1-(1-isobutyryloxy-éthoxycarbonylamino)-éthyl]-phosphinoylméthyl}-1-phényl-pipéridine-4-carbonyl)-amino]-propionique
Acide 2-[(1-Benzenesulfonyl-4-{hydroxy-[1-(1-isobutyryloxy-éthoxycarbonylamino)-éthyl]-phosphinoylméthyl}-pipéridine-4-carbonyl)-amino]-propionique

Les composés de formule (I) de l'invention possèdent potentiellement 3 centres d'asymétrie, à savoir les carbones portant les radicaux respectifs R₂, R₅ et R₆ interagissant avec le site actif des deux enzymes. De manière avantageuse, ces 3 centres asymétriques sont résolus et de configuration absolue respective optimisant les propriétés des composés de l'invention, à savoir :
- l'atome de carbone portant le radical R₂ est de configuration R (excès énantiomérique supérieur à 90%) ;
- le cas échéant, l'atome de carbone portant le radical R₅ est de configuration S (ee>90%) ;
- l'atome de carbone portant le radical R₆ est de configuration S (ee>90%).

Le cas échéant, la configuration de l'acide phosphinique est laissée libre (on peut avoir un énantiomère, l'autre énantiomère ou un mélange racémique).

Dans ce qui suit, nous décrivons la synthèse des composés selon l'invention pour lesquels la configuration des 3 atomes de carbone asymétriques est résolue et est de configuration absolue respective optimisant les propriétés des molécules (ee>90%). S'il le souhaite, l'homme du métier sait adapter le procédé décrit pour obtenir toute configuration désirée.

Les composés de formule (Ia) pour lesquels R₅=H, R₃=H, et R₇#H sont obtenus par condensation des composés VI avec des α-aminoesters VII selon le schéma suivant :

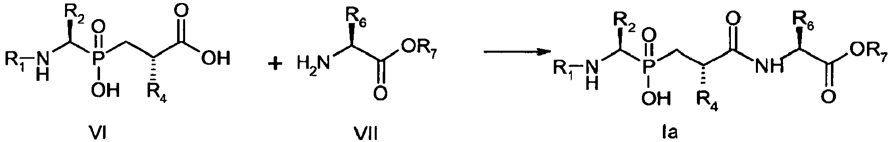

Cette réaction est réalisée par action d'un agent de couplage tel que le chlorhydrate de 1-(3-dimethylaminopropyl)-3-éthylcarbodiimide (EDC) ou le 2-(1H-benzotriazole-1-yl)-1,1,3,3-tetramethyluronium tetrafluoroborate (TBTU), en présence notamment d'une amine tertiaire telle que la diisopropyléthylamine (DIEA). Le diméthylformamide (DMF) est utilisé avantageusement comme solvant dans cette étape.

Les composés pour lesquels R₅=H, R₃=H et R₇=H, pourront être préparés par hydrogénolyse d'un composé de formule (Ia) pour lequel R₇=Bn, par des techniques bien connues de l'homme du métier, notamment en présence d'un catalyseur tel que le palladium sur charbon sous atmosphère d'hydrogène.

Les composés aminophosphiniques (VI) sont obtenus en trois étapes à partir des composés (II) et (III) : 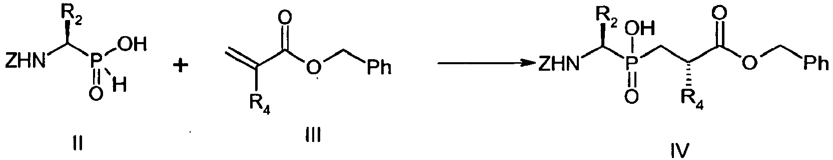 Z représente un groupement benzyloxycarbonyle.

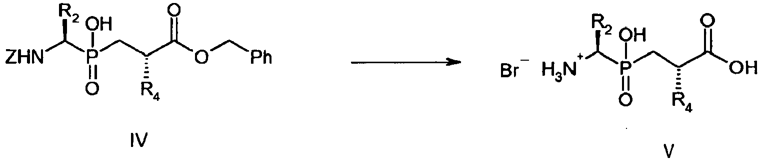

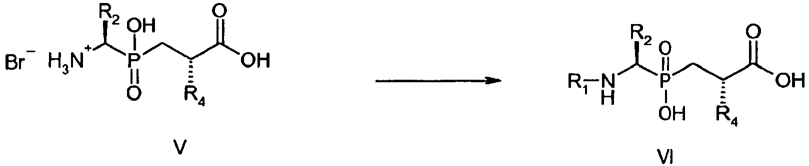

La première étape consiste à condenser les composés (II) et (III) en présence de bis-triméthylsilyle acétamide pour donner le composé (IV). La réaction est effectuée avantageusement à une température comprise entre 70 et 120°C, sans solvant.

Le composé (IV) est obtenu sous forme d'un mélange de deux diastéréoisomères (2R, 4S)/(2R, 4R) dans des proportions d'environ 65/35. Le stéréoisomère (2R, 4S) majoritaire est alors séparé du stéréoisomère (2R, 4R) par précipitation dans un solvant organique tel que l'acétate d'éthyle, l'éther diéthylique, l'isopropanol, l'acétonitrile ou un mélange de ceux-ci, et de préférence dans l'acétate d'éthyle, suivi ou non d'une purification supplémentaire par recristallisation.

Le composé (IV) ainsi obtenu est alors successivement déprotégé en position C-terminale par saponification (NaOH) et en N-terminale par action de HBr/CH₃CO₂H pour donner un composé (V). Le composé (V) est alors condensé avec un acyloxyalkyl(*p*-NO₂-phenyl) carbonate, préparé selon Alexander et al., 1988, J. Med. Chem., 31,318, ou avec un acyloxyalkylsuccinyl carbonate, préparé selon Sun et al. Bioorg. Med. Chem. Lett. 2001, 11, 1875, dans le dioxane en présence de NaHCO₃.

De manière alternative, les composés (Ia) peuvent être synthétisés par condensation de (VII) sur l'intermédiaire (XI) (Z-NH-CH(R₂)P(O)OH-CH₂CH(R₄)COOH) obtenu par hydrolyse alcaline de l'ester benzylique de (IV), dans les conditions de couplage décrites ci-dessus (EDCI ou TBTU en présence de DIEA dans le DMF). On obtient alors, le composé (XII), qui après déprotection du groupement Z conduit au composé (XIII). Celui-ci conduit à (Ia) par les procédés décrits précédemment lors de la transformation de (V) en (VI).

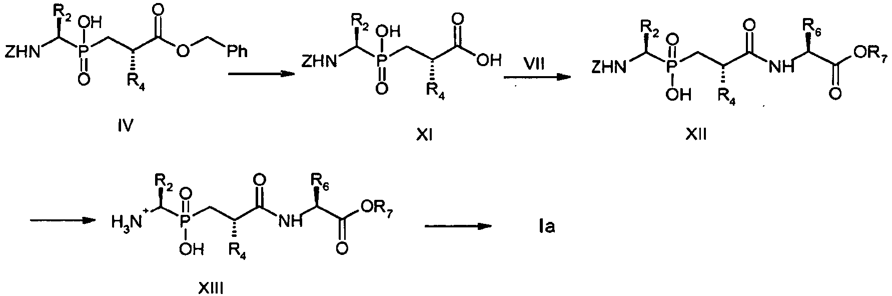

Les composés (II) optiquement purs, de configuration (R) sont préparés à partir des aldéhydes (VIII) en suivant le protocole de synthèse et la méthode de résolution décrits par Baylis et al. dans J. Chem. Soc. Perkin Trans I (1984), 2845. 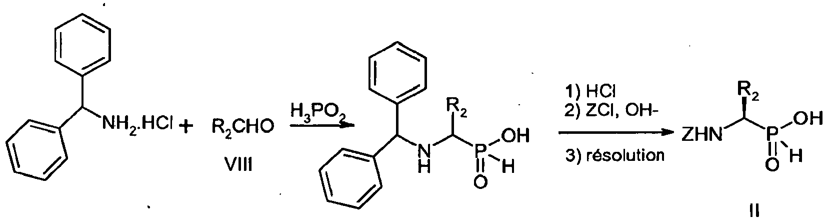 Z représente un groupement benzyloxycarbonyle.

L'ester benzylique des acrylates (III) est préparé à partir des acides acryliques correspondants (X) par action du bromure de benzyle en présence de K₂CO₃ dans l'acétonitrile à 80°C. Les acides (X) sont obtenus par un protocole classique, bien connu de l'homme du métier, qui comprend les étapes suivantes: condensation d'un aldéhyde (IX) sur le diéthylmalonate (étape 1), réduction de la double liaison et saponification des fonctions esters (étape 2 : 1)NaBH₄, 2)OH⁻, 3)H⁺) et réaction de Mannich (étape 3).

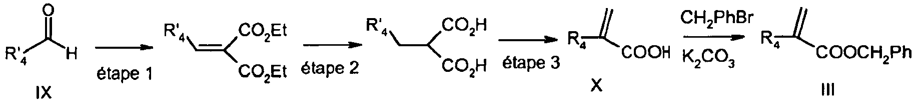

Les composés de formule (Ib), pour lesquels R₅≠H et R₃=H, sont obtenus par le même couplage que celui décrit pour les composés (Ia) en remplaçant l'intermédiaire (VI) par son analogue (VIbis).

Celui-ci est obtenu par condensation de l'acide aminophosphinique protégé (II bis) et d'un alcool activé sous forme de mésylate ou de triflate (XIV) en présence de lithium diisopropyl amide (LDA) (McKittrick et al. dans Bioorg. Med. Chem. Lett. (1996), 1629) 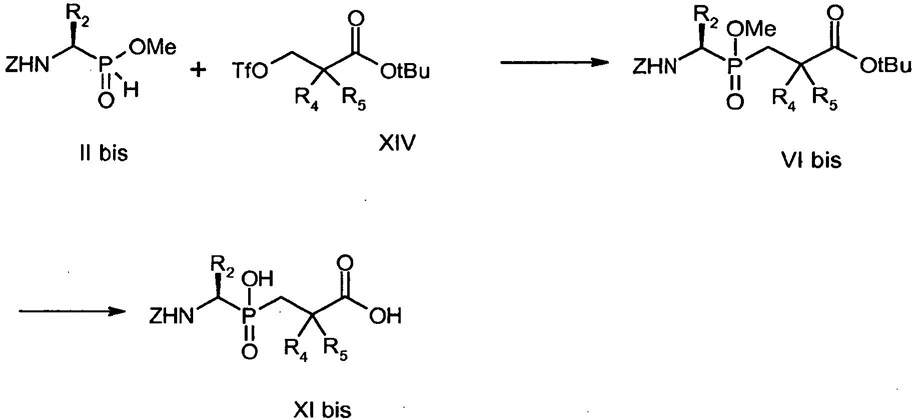 Z représente un groupement benzyloxycarbonyle.

Les composés (XIV) sont préparés à partir de l'acide carboxylique correspondant de formule CH(R₄)(R₅)-CO₂H. Après estérification sous forme d'ester t-butylique pour donner CH(R₄)(R₅)-CO₂tBu (XV), le traitement par le paraformaldéhyde en présence de Lithium diisopropylamide (iPr₂NLi)conduit à l'ester alcool HOCH₂-C(R₄)(R₅)-COOtBu. La fonction alcool est alors activée sous forme de mésylate ou de triflate pour conduire au composé (XIV). 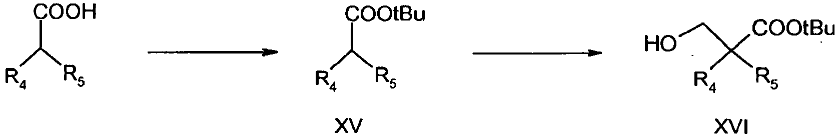 La déprotection des esters tButylique de l'acide carboxylique et méthylique de l'acide phosphinique du composé (VI bis) sont déprotégés par le TFA dans le chlrorure de méthylène pour conduire au composé (XI bis). La fin de la synthèse est conduite comme décrit précédemment.
Les composés de formule (Ic) pour lesquels R₃#H et R₇#H sont préparés à partir des composés (Ia) et (Ib) correspondants pour lesquels R₃=H. L'alkylation de la fonction phosphinique est obtenue par action d'un halogéno(acyloxy)alkyle (l'halogène est un chlore ou un brome). Cette alkylation est réalisée en présence de sulfate de tetrabutylammonium, de DIEA, et de NaI dans un solvant comme le toluène ou le chloroforme, de préférence le toluène. La déprotection éventuelle de la fonction carboxylate (R₇=H) est obtenu comme précédemment par hydrogénolyse de l'ester benzylique (R₇=CH₂Ph).

De manière plus générale, la présente invention a pour objet un procédé de préparation d'un composé de formule (I) tel que défini ci-dessus, selon les étapes suivantes :
- couplage peptidique entre un composé de formule (A) suivante :

   R₁-NH-CH(R₂)-P(=O)(OH)-CH₂-C(R₄)(R₅)-COOH (A),

   et un composé de formule (B) suivante :

   H₂N-CH(R₆)-COOR₇ (B),

   pour donner un composé (I) dans laquelle R₃=H et R₁, R₂, R₄, R₅, R₆ et R₇ sont tels que définis ci-dessus, R₇ ne représentant toutefois pas un atome d'hydrogène ;
- éventuellement une étape de substitution de la fonction acide phosphonique par un groupe R₃#H,
- éventuellement une étape d'hydrolyse (saponification ou hydrogénolyse) de la fonction -COOR₇ ;
- séparation du milieu réactionnel du composé (I) obtenu à l'une des étapes précédentes.

Ce procédé pourra être suivi par d'éventuelles réactions supplémentaires de substitution et/ou protection/déprotection bien connues de l'homme du métier. L'étape de séparation du milieu réactionnel pourra être réalisée par des méthodes bien connues de l'homme du métier, comme par exemple par extraction, évaporation du solvant ou encore par précipitation et filtration. Le composé ainsi obtenu pourra alors être purifié si nécessaire par des techniques bien connues de l'homme du métier, comme par recristallisation si le composé est cristallin, par distillation, par chromatographie sur colonne sur gel de silice ou encore par chromatographie liquide haute performance (CLHP).

La présente invention a également pour objet un composé de formule (I) tel que défini ci-dessus pour son utilisation en tant que médicament, notamment destiné au traitement de la douleur et en particulier (1) des douleurs neuropathiques ou neuroinflammatoires ou (2) des douleurs vives (en particulier les douleurs aiguës).

Par « douleurs neuropathiques ou neuroinflammatoires », on entend plus particulièrement, mais de manière non limitative, des douleurs causées par un diabète sucré de type I ou II, une infection virale ou rétrovirale, une chimiothérapie d'un cancer, une radiothérapie, une intervention chirurgicale incluant l'illusion des amputés et les séquelles d'une mastectomie, l'alcoolisme, une névralgie faciale, un traumatisme tel qu'un syndrome du plexus brachial, une radiculopathie ou une radiculagie telle qu'une sciatique, une cruralgie ou un syndrome du défilé thoracobrachial, une fibromyalgie, un syndrome des jambes sans repos, une douleur articulaire inflammatoire causée notamment par l'arthrite ou une phase aigue de l'arthrose, une douleur articulaire dégénérative causée notamment par l'arthrose, ou encore une lombalgie.

Ces douleurs neuropathiques et neuroinflammatoires sont caractérisées par : i) un excès de nociception dénommé « hyperalgie », évaluée par des tests appropriés sur des modèle animaux prédictifs tels que le Plantar test ou la plaque chaude ; ii) une allodynie qui correspond à une sensation douloureuse provenant d'une région qui n'est pas concernée par le stimulus nociceptif original et dont la réduction est mesurée en particulier par le test de Von Frey.

La douleur vive provient de la stimulation de récepteurs, canaux ou autres cibles qui transmettent au cerveau un message interprété comme étant une douleur aiguë. Par « douleurs vives », on entend désigner en particulier les douleurs post-opératoires, la douleur chez une personne atteinte du cancer, des douleurs dues à des lésions des tissus périphériques provoquant un excès d'influx douloureux dans le système nerveux. On désigne en particulier les brûlures, les traumatismes, les suites d'une opération et d'un grand nombre de maladies, entrainant soit des douleurs aiguës (pathologies postopératoire, traumatique, infectieuse, dégénérative), soit des douleurs chroniques (pathologies lésionnelles persistantes plus ou moins évolutives).

L'invention concerne également l'utilisation d'un composé de l'invention pour la fabrication d'un médicament destiné au traitement de la douleur et en particulier (1) des douleurs neuropathiques ou neuroinflammatoires ou (2) des douleurs vives. L'invention concerne également une méthode pour le traitement de la douleur et en particulier (1) des douleurs neuropathiques ou neuroinflammatoires ou (2) des douleurs vives, comprenant l'administration d'une quantité efficace d'au moins un composé de l'invention à un patient en ayant besoin.

La présente invention a également pour objet une composition pharmaceutique comprenant au moins un composé de formule (I) tel que défini ci-dessus et au moins un véhicule pharmaceutiquement acceptable. Les composés selon l'invention peuvent être administrés par voie orale, sublinguale, parentérale, sous-cutanée, pulmonaire, nasale, intramusculaire, intraveineuse, intrathécale, intra-articulaire ou transdermique. L'ingrédient actif peut être administré sous formes unitaires d'administration, en mélange avec des supports pharmaceutiques classiques, aux animaux ou aux êtres humains. Cette composition peut comprendre, dans la même formulation ou en association sous une forme différente, au moins un autre principe actif, en particulier un analgésique choisi dans le groupe constitué par la morphine et ses dérivés, le tétrahydrocannabinol et ses dérivés, les dérivés du Gaba (Gabapentine ou Prégabaline), les toxines botuliques (toxine botulique A) ou un antagoniste des récepteurs purinergiques (récepteur P2X3).

La présente invention décrit également une composition pharmaceutique comprenant :
(i) au moins un composé de formule (I) tel que défini ci-dessus, et
(ii) au moins un autre principe actif, choisi dans le groupe constitué par la morphine et ses dérivés, le tétrahydrocannabinol et ses dérivés, les dérivés du Gaba (Gabapentine ou Prégabaline), les toxines botuliques (toxine botulique A) et un antagoniste des récepteurs purinergiques (récepteur P2X3), en tant que produits de combinaison pour une utilisation simultanée, séparée ou étalée dans le temps.

Ces compositions et les composés de formule (I) peuvent être utilisés en tant que médicaments.

Selon une première variante avantageuse de l'invention, les composés sont destinés au traitement de douleurs neuropathiques ou neuroinflammatoires et sont administrés par voie orale.

La composition pharmaceutique sera donc de préférence formulée pour une administration par voie orale. Les formes unitaires d'administration appropriées comprennent les comprimés, les gélules, les poudres, les granules et les solutions ou suspensions orales; on mélange l'ingrédient actif principal avec un véhicule pharmaceutique approprié. La formulation peut être de telle sorte qu'elle ait une activité prolongée ou retardée et qu'elle libère d'une façon continue une quantité prédéterminée de principe actif

Les inventeurs ont constaté que les composés, administrés par voie orale, ne traversent pas la barrière hématoencéphalique (BHE) et ainsi ne pénètrent pas dans le cerveau. Dans ces conditions, les éventuels effets secondaires non désirables, même faibles, des opioïdes endogènes cérébraux (enképhalines) (F. Noble et B.P. Roques, Exp. Op. Ther. Targets, 2007, 11, 145-159) qui pourraient provenir de l'activation des récepteurs opioïdes du cerveau, par ces enképhalines protégées de leur inactivation par les composés de l'invention, sont totalement exclus.

Cette composition peut comprendre au moins un autre principe actif. En particulier, cette composition pharmaceutique pourra comprendre un analgésique choisi dans le groupe constitué par les dérivés du Gaba tels que la Gabapentine ou la Prégabaline, ou un antagoniste du récepteur purinergique P2X3 et les toxines botuliques telles que la toxine botulique A. Par exemple, les composés de formule (I) formulés pour une administration par voie orale, peuvent être utilisés en association avec une toxine botulique injectée localement.

Selon une seconde variante avantageuse de l'invention, les composés sont destinés au traitement de douleurs nociceptives et sont administrés par une voie autre que par la voie orale comme par exemple par voie sublinguale, parentérale, sous-cutanée, nasale, pulmonaire intramusculaire, intraveineuse, intrathécale, intra-articulaire ou transdermique, en particulier par voie intraveineuse. L'ingrédient actif peut être administré sous formes unitaires d'administration, en mélange avec des supports pharmaceutiques classiques, aux animaux ou aux êtres humains. La composition pharmaceutique sera donc formulée pour une administration par voie intraveineuse.

La présente invention décrit également l'utilisation en association (en tant que produits de combinaison pour une utilisation simultanée, séparée ou étalée dans le temps) avec un autre analgésique tel que la morphine et ses dérivés, le tétrahydrocannabinol et ses dérivés, ou un antagoniste du récepteur purinergique P2X3.

Selon l'une quelconque des variantes envisagées, les composés de l'invention peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 0,01 mg et 100 mg, encore plus avantageusement entre 0,1 mg et 10 mg.

### FIGURES

### Légende des figures :

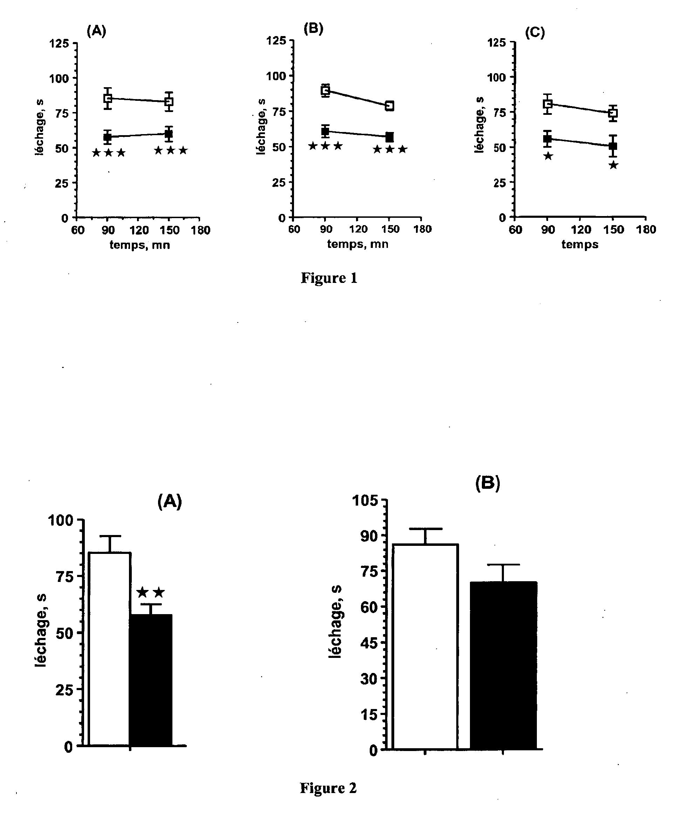

Dans toutes les figures, les analyses statistiques (p, test de Student) sont indiquées de la manière suivante :
★ p < 0,1 versus contrôle
★ ★ p < 0,01 versus contrôle
★ ★ ★ p < 0,001 versus contrôle
   La **figure 1** représente le temps total de lèchement (exprimé en secondes pendant la période d'examen de 5 min) de la patte d'une souris qui a reçu de la formaline en fonction du temps (minutes) après administration par voie orale du véhicule (□) ou d'un composé selon l'invention (■), les graphes (A), (B) et (C) correspondant respectivement aux composés des exemples 8, 3 et 4.
   La **figure 2** représente le temps total de lèchement (exprimé en secondes pendant la période d'examen de 5 min) de la patte d'une souris traitée par la formaline, 90 mn après administration par voie orale : (A) du véhicule (□) ou du composé 8 (50 mg/kg) selon l'invention (■) ou (B) du véhicule (□) ou de la molécule de référence (100 mg/kg) (■).

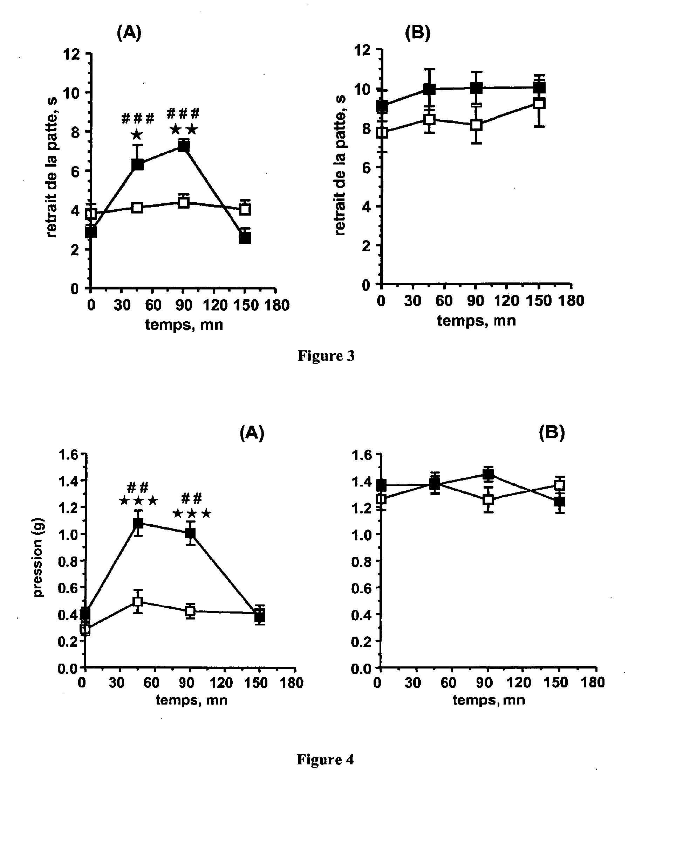

   La **figure 3** représente la réponse obtenue dans un modèle de douleur neuropathique par ligature partielle et unilatérale du nerf sciatique d'une souris (Plantar test), permettant l'évaluation de l'hyperalgie thermique. Après le retrait de la patte est mesuré (en secondes) en fonction du temps (en minutes) 14 jours après la chirurgie. Le graphe (A) représente la réponse observée pour la patte ipsilatérale, tandis que le graphe (B) représente la réponse obtenue pour la patte contralatérale.
      (moyenne des pattes contralatérales à 90 mn : véhicule (7,85), composé 3 = 9,4 s)
   La **figure** 4 représente la réponse obtenue dans un test de von Frey (mesure de l'allodynie) à une pression (en g) de la patte d'une souris à l'aide de filaments de dureté croissante, en fonction du temps (mn) 14 jours après la chirurgie et après administration par voie orale du véhicule (□) ou du composé de l'exemple 3 (50 mg/kg) (■). Le graphe (A) représente la réponse observée pour la patte ipsilatérale, tandis que le graphe (B) représente la réponse obtenue pour la patte contralatérale.

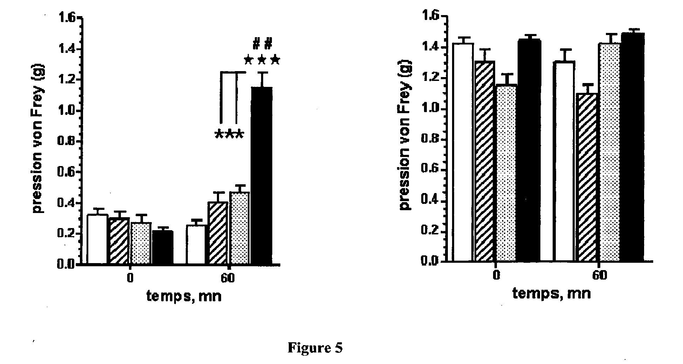

   La **figure 5** représente les réponses obtenues à 60 min dans un test de Von Frey, comme décrit dans la légende de la figure 4. Le composé de l'exemple 4 est administré seul p.o. à la dose de 10 mg/kg ( 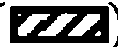 ) et la gabapentine, également p.o. à la dose de 30 mg/kg ( 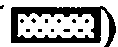 ). Ces deux composés sont administrés p.o., en association, aux doses précédentes ( 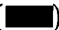 ). Le contrôle ( 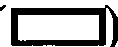 ) correspond à l'administration p.o. du véhicule.

### EXEMPLES

Les exemples suivants permettent d'illustrer l'invention sans pour autant la limiter. Les abréviations suivantes ont été utilisées :
- CCM: Chromatographie sur Couche Mince
- CLHP: Chromatographie liquide haute performance
- DMSO: Diméthylsulfoxyde
- éq.: Equivalent
- ESI: Ionisation par électrospray
- mn: Minutes
- RMN: Résonance Magnétique Nucléaire
- TFA: Acide trifluoroacétique

### Exemple 1 : Acide (R)(1-Benzyloxycarbonylamino-éthyl)-phosphinique

### Etape 1 : Acide (1-((diphénylméthyl)amino)-éthyl)phosphinique

Un mélange de 200g (0,91 mole) de chlorhydrate de diphénylméthylamine dans 600mL d'eau et de 132 mL (1,0 mole) d'acide phosphinique (50% dans l'eau) est porté au reflux sous agitation. On ajoute 56 mL (1,0 mole) d'acétaldéhyde en solution dans 350 mL d'eau, goutte à goutte en 30 mn.

La réaction est suivie par CLHP. Après 2 h, la réaction est ramenée à température ambiante. Le précipité blanc obtenu est filtré, lavé à l'eau (2x300 mL) et à l'acétone (2x300 mL) puis séché. Solide blanc, 225 g (90%)

### Etape 2 : Acide (1-aminoéthyl)-phosphinique

Un mélange de 225 g du composé de l'étape 1 et de 2L d'HCl 6N est porté au reflux pendant 5h. Après refroidissement, le milieu réactionnel est concentré de moitié puis extrait par 3x1,5 L d'éther éthylique. La solution est évaporée à sec et le résidu huileux (130 g) est repris par 1,3L d'éthanol. La solution est refroidie à 0°C et on ajoute 450 mL d'oxyde de propylène. Un solide blanc précipite. Le précipité est essoré, lavé à l'éthanol (2x100 mL), à l'éther éthylique (2x100 mL) et séché. Solide blanc, 65 g (73%)
RMN (1H) DMSO d6 δ (ppm): 1,26 (3H d,d); 3,32 (1H m); 6,98 (1H d); 8,23 (3H s).

### Etape 3 : Acide (R)(1-benzyloxycarbonylamino-éthyl)-phosphinique

On solubilise, dans 300mL d'eau, 65g (0,59 mole) du composé de l'étape 2 et le pH est ajusté à 9,5 par ajout de soude. On ajoute sous agitation 85 mL de chloroformiate de benzyle. Après 1h à 0°C, le mélange est ramené à température ambiante et est versé sur un mélange de glace (IL) et d'HCl concentré (300 mL). Le précipité blanc formé est essoré, lavé à l'eau (2x100 mL) et séché. Solide blanc, 131 g (91%) CLHP (CH₃CN (0,1 % TFA)/ H₂0 (0,1% TFA) 30/70, Colonne Kromasil C18), Rt 4,6mn.

La résolution de l'acide phosphinique racémique est effectuée par recristallisation du sel obtenu avec la (R) (+) α-methylbenzylamine, comme décrit dans Baylis et al. (J. Chem. Soc. Perkin Trans I 1984, 2845) dans un mélange acétate d'éthyle/isopropanol=3,5/1. Solide blanc, 48 g (86%)
RMN (1H) DMSOd6 δ (ppm) : 1,19 (d,3H); 3,66(m,1H); 5,03 (s,2H); 6,78 (d,1H); 7,35 (s,5H); 7,62 (d,1H)

### Exemple 2: Ester benzylique de l'acide 2-biphényl-4-ylméthyl-acrylique

### Etape 1 : Acide 2-biphényl-4-ylméthyl-malonique

Le protocole utilisé est celui décrit dans Organic Synthesis Coll. Vol.3 p 337. A partir de 48,4 g de diéthylmalonate et 50 g de diphényl-4-carboxaldéhyde, on obtient 88,2 g de diéthyl ester de l'acide 2-biphényl-4-ylméthylène-malonique.

Ce composé (88 g) est mis en solution dans l'éthanol (500mL) et la double liaison est réduite par action du borohydrure de sodium (10,3 g). Après 1 h, la réduction est terminée et le mélange réactionnel est dilué dans l'éthanol (500mL) et 1,36 L de NaOH 1N sont ajoutés à 0°C.

Après évaporation de l'éthanol et acidification de la phase aqueuse, on obtient l'acide 2-biphényl-4-ylméthyl-malonique sous forme d'un solide blanc, 64,8 g (88%). CLHP (CH₃CN (0,1 % TFA)/ H₂0 (0,1% TFA) 70/30, Colonne Kromasil C18), Rt 3,5 mn.

### Etape 2 : Acide 2-biphényl-4-ylméthyl-acrylique

Le diacide obtenu à l'étape 1 (60 g) est solubilisé dans 300 mL de THF et 23,5 mL (2éq.) de diéthylamine et 19,5 mL (4éq.) de formaldéhyde sont ajoutés. Le milieu réactionnel est mis au reflux sous agitation pendant 20h. Après refroidissement, le THF est évaporé et le résidu est repris dans l'acétate d'éthyle (500mL) et acidifié par 100mL d'HCl 6N. La phase organique est récupérée, lavée par 2x350 mL d'eau 1x300mL de NaCl sat. et séchée sur Na₂SO₄. On obtient une poudre blanche, 51 g (97%).
CLHP (CH₃CN (0,1 % TFA)/ H₂0 (0,1% TFA) 70/30, Colonne Kromasil C18), Rt =6,2 mn.

### Etape 3 : Ester benzylique de l'acide 2-biphényl-4-ylméthyl-acrylique

L'acide 2-biphényl-4-ylméthyl-acrylique (30 g) est mis en suspension dans 300 mL d'acétonitrile. On ajoute 21 g de K₂CO₃ puis 16,5 g de bromure de benzyle et on chauffe à 80°C pendant 15 h. Le milieu réactionnel est évaporé et le résidu repris dans l'acétate d'éthyle. La phase organique est lavée avec une solution de Na₂CO₃, à l'eau et avec une solution aqueuse saturée de NaCl, puis séchée sur Na₂SO₄. Après filtration, la phase organique est évaporée à sec. Produit huileux, 40g (96%).
CLHP (CH₃CN (0,1 % TFA)/ H₂O (0,1% TFA) 80/20, Colonne Kromasil C18), Rt =12,7 mn.
RMN (CDCl₃) δ (ppm) :3,6 (2H s); 5,1 (2H S); 5,5 (1H d); 6,2 (1H d); 7,1-7,5 14H m).

### Exemple 3: Ester benzylique de l'acide 2-(2-biphényl-4-ylméthyl-3-{hydroxy-[1-(1-isobutyryloxy-éthoxycarbonylamino)-éthyl]-phosphinoyl}-propionylamino)-propionique

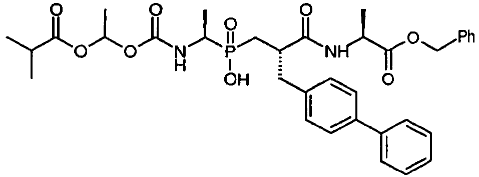

### Etape 1 : Ester benzylique de l'acide 3-[(2-benzyloxycarbonyl-3-biphényl-4-yl-propyl)-hydroxy-phosphinoyl]-butyrique.

Le synthon aminophosphinique de l'exemple 1 (9,6 g) et l'ester benzylique de l'acide 2-biphényl-4-ylméthyl-acrylique de l'exemple 2, étape 3(14,3 g) sont additionnés à 21mL de bis-trisméthylsilylacetamide (BSA) et le mélange réactionnel est agité 5 h à 70°C. Après refroidissement, le mélange est dilué dans l'acétate d'éthyle. On ajoute quelques gouttes d'eau jusqu'à formation d'un précipité. Le solide en suspension est agité pendant 1h à température ambiante, puis est essoré, lavé à l'acétate d'éthyle et séché sous vide. On obtient 11,6 g (79%) du diastéréoisomère de configuration (R)(S).
CLHP (CH₃CN (0,1 % TFA)/ H₂O (0,1% TFA) 55/45, Colonne Kromasil C18) Rt=17,8 min.
RMN 1H (DMSO) d6 δ (ppm) 1,26 (3H dd); 1,81-2,16 (2H m); 2,78-3,12 (3H m); 3,78 (1H qt); 4,94-5,08 (2x2H 2q); 7,12-7,68 (19H arom.) +NH (m).

### Etape 2: Bromhydrate d'ammonium de 1-[(3-biphényl-4-yl-2-carboxy-propyl) hydroxy-phosphinoyl]-éthyl

Le composé de l'étape 1 (10,6g) est mis en suspension dans le THF (100mL) et 7,4 g de soude en solution dans 50 mL d'eau sont ajoutés sous agitation à 0°C. Le mélange est agité à température ambiante pendant une nuit. Le THF est évaporé, la phase aqueuse est acidifiée à pH 1 par HCl 1N, puis extraite par l'acétate d'éthyle. La phase organique est séchée sur Na₂SO₄, filtrée et évaporée à sec. Solide blanc, 7,4 g (83%).

L'acide ainsi obtenu (6 g) est mis en solution dans 60 mL d'acide bromhydrique (45% dans CH₃CO₂H) et la solution est agité 1h à température ambiante.

Le mélange réactionnel est ensuite évaporé à sec et séché sous vide pour conduire à un produit gommeux orangé, 6,9g (99%)

### Etape 3 : Acide 2-biphényl-4-ylméthyl-3-{hydroxy-[1-(1-isobutyryloxy éthoxycarbonylamino)-éthyl]-phosphinoyl}-propionique

Le composé de l'étape 2 (6,23 g) est mis en suspension dans l'acétonitrile (70 mL). On ajoute successivement 9,8g (8éq.) de NaHCO₃ en solution dans 70 mL d'eau et 4,76 g (1,1 éq.) d'ester 1-[2-(4-nitro-phenyl)-acetoxy]-ethyl de l'acide isobutyrique. Le mélange est agité à température ambiante pendant une nuit.

Après évaporation de l'acétonitrile, la phase aqueuse est extraite par l'acétate d'éthyle. La phase organique est lavée par de l'acide citrique à 10%, une solution aqueuse saturée de NaCl, séchée sur Na₂SO₄, filtrée et évaporée à sec. Le produit brut est chromatographié sur gel de silice en utilisant le mélange CH₂Cl₂/MeOH 9/1 puis 7/3 comme éluant. Solide blanc, 4,42 g (60%).
CLHP (CH₃CN (0,1 % TFA)/ H₂O (0,1% TFA) 50/50, Colonne Kromasil C18), Rt= 3,7 mn.
RMN (DMSO d6) δ ppm : 0,95-1,45 (12H m); 1,63-2,15 (2H m); 2,45 (1H m); 2,81-3,03 (3H m); 3,68 (1H m); 6,63 (1H m); 7,20-7,82 (10H m).

### Etape 4 : Ester benzylique de l'acide 2-(2-biphényl-4-ylméthyl-3-{hydroxy-[1-(1-isobutyryloxy-éthoxycarbonylamino)-éthyl]-phosphinoyl}-propionylamino)-propionique

Le composé de l'étape 3 (500mg) est mis en solution dans le diméthylformamide (DMF) (10 mL) et on ajoute successivement 945 mg (3éq.) de TBTU, 1 mL de DIEA (6éq.) et 257 mg (1,2éq.) de chlorhydrate de l'ester benzylique de l'alanine. Le mélange est agité à température ambiante pendant 15 mn, puis DMF est évaporé. Le résidu est repris par l'acétate d'éthyle, puis successivement lavé avec une solution d'acide citrique à 10%, de l'eau, une solution de NaHCO₃ à 10%, une solution aqueuse saturée de NaCl et est séché sur Na₂SO₄. Après filtration, la solution est évaporée sous vide. Solide blanc, 1 g (95%).
CLHP (CH₃CN (0.1 % TFA)/ H₂0 (0.1% TFA) 50/50, Colonne Kromasil C18) Rt= 8,2 mn.
RMN 1H (DMSOd6) δ (ppm) : 0,98-1,42 (15H m); 1,48-1,82 (2H m); 2,45 (1H m); 2,69-3,05 (3H m); 3,68 (1H m); 4,33 (1H m); 5,06 (2H s); 6,62 (1H m); 7,21-7,67 (15H m); 8,45 (1H t).

### Exemple 4 : Acide 2-(2-biphényl-4-ylméthyl-3-{hydroxy-[1-(1-isobutyryloxy-éthoxycarbonylamino)-éthyl]-phosphinoyl}-propionylamino)-propionique

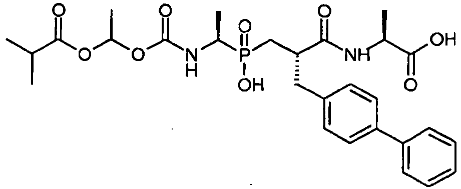

On solubilise 250 mg du composé de l'exemple 3 dans 10 mL de MeOH et on ajoute 125 mg de Pd/C 10%. Le mélange est hydrogénolysé à température et pression ordinaire sous atmosphère d'hydrogène. Au bout d'1h, on ajoute 20 mL de CH₂Cl₂ et on filtre sur Célite. On évapore à sec et on sèche sous vide. Solide blanc 184 mg (89%).
CLHP (CH₃CN (0,1 % TFA)/ H₂O (0,1% TFA) 60/40, Colonne Kromasil C18) Rt= 4,1mn.
Masse ESI(+) [M+H]⁺=577.
RMN (1H) DMSO d6, δ (ppm) : 1-1,5 (15 H m); 1,5-2,0 (2H m); 2,5 (1H, m); 3,0 (3H m); 3,7 (1H m); 4,2 (1H m); 6,2 (1H m); 7,3-7,7 (9H+1H m); 8,4 (1H dd).

### Exemple 5 : Sel de sodium de l'acide 2-(2-biphényl-4-ylméthyl-3-{hydroxy-[1-(1-isobutyryloxy-éthoxycarbonylamino)-éthyl]-phosphinoyl}-propionylamino)-propionique

87 mg du composé de l'exemple 4 sont dissous dans un mélange de 1 mL d'eau et 1 mL d'acétonitrile. On ajoute 12,6 mg de NaHCO₃. La solution obtenue est lyophilisée et conduit à 87 mg du composé attendu.

### Exemple 6 : Ester éthylique de l'acide 2-(2-biphényl-4-ylméthyl-3-{hydroxy-[1-(1-isobutyryloxy-éthoxycarbonylamino)-éthyl]-phosphinoyl}-propionylamino)-propionique

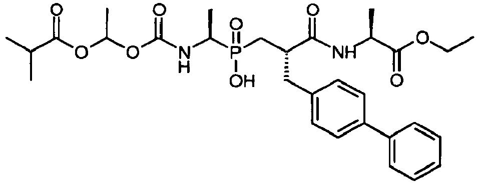

Le composé de l'exemple 3 (500 mg) est couplé dans les conditions de l'étape 4 de l'exemple 3 avec du chlorhydrate de l'ester éthylique d'alanine (152 mg). Le produit brut obtenu est chromatographié sur gel de silice en utilisant le mélange CH₂Cl₂/MeOH/AcOH 7/3/0.2. Solide blanc, 530 mg (88%).
Masse ESI(+) [M+H]⁺=605
RMN 1H (DMSO d6) δ (ppm): 1,0-1,5 (18H m); 1,6-1,9 (2H m); 2,5 (1H m); 2,7-3,0 (3H m); 3,7 (1H m); 4,0 (2H q); 4,2 (1H qt); 6,6 (1H m); 7,2-7,7 (9H+1H m); 8,4 (1H dd).

### Exemple 7 : Trifluoroacétate de l'ester 1-éthoxycarbonyloxyéthyle de l'alanine

### Etape 1 : 1-Ethoxycarbonyloxyéthyl ester de la N-terbutyloxycarbonyl-L-alanine

La N-boc-L-alanine (boc = tert-butyloxycarbonyle) (10 g) est mise en solution dans 100 mL d'acétate d'éthyle en présence de triéthylamine (6,4 g ,1,2 éq.) sous atmosphère inerte et le mélange est agité 15 mn à température ambiante. On ajoute ensuite NaI (1,9 g, 0,2 éq.) et le 1-chloroéthyléthylcarbonate (9,38 g , 1,1 éq.). Le mélange est porté au reflux pendant 15 h. Lorsque la réaction est complète, le mélange est lavé par une solution aqueuse deNaHCO₃ à 10% (2 fois), H₂O, et une solution aqueuse saturée de NaCl. La phase organique est séchée sur Na₂SO₄, filtrée et évaporée à sec. Huile légèrement jaune. 12,9g (80%)
CCM (cyclohexane/EtOAc :6/4) Rf=0,73.

### Etape 2 : Trifluoroacétate de l'ester 1-éthoxycarbonyloxyéthyl de la L-alanine

Le composé de l'étape 1 (12,9 g) est solubilisé dans 25 mL CH₂Cl₂ et 25 mL d'acide trifluoroacétique sont ajoutés à 0°C. Le mélange est agité à température ambiante pendant 3h. Lorsque la réaction est totale, le milieu réactionnel est évaporé à sec. Le résidu est repris 3 fois avec du cyclohexane et le mélange est ré-évaporé pour éliminer l'excès de TFA. Huile légèrement brune, 13,4 g (99%).
RMN 1H (DMSOd6) δ (ppm) :1,2 (3H t); 1,35 (3H dd); 1,45 (3H d); 4,1 (2H q); 6,7 (1H m); 8,3 (3H s).

### Exemple 8 : Ester 1-ethoxycarbonyloxy-ethylique de l'acide 2-(2-biphényl-4-ylméthyl-3-{hydroxy-[1-(1-isobutyryloxy-éthoxycarbonylamino)-éthyl]-phosphinoyl}-propionylamino)-propionique

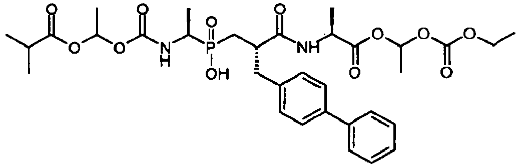

Le composé de l'exemple 3 (500 mg) est couplé, dans les conditions de l'étape 4 de l'exemple 3, avec le composé de l'exemple 7, (500 mg, 1,2 éq.)). Le produit brut est chromatographié sur gel de silice (CH₂Cl₂/MeOH/AcOH :7/3/0,2. Solide blanc, 330mg (55%).
CLHP (CH₃CN (0,1 % TFA)/ H₂O (0,1% TFA) 70/30, Colonne Kromasil C18) Rt=5,4 mn.
Masse ESI(+) [M+H]⁺=693
RMN 1H (DMSOd6) δ (ppm) : 1,0-1,4 21Hm); 1,5-2,0 (2H m); 2,5 (1Hm); 2,7-3,0 (3H m); 3,65 (1H m), 4,15 (2H q); 4,25 (2H m); 6,6 (2H m); 7,2-7,7 (9H+1H m); 8,5 (1H dd).

### Exemple 9: Acide 2-(2-biphényl-4-ylméthyl-3-{(1-isobutyryloxy-éthoxy)-[1-(1-isobutyryloxy-éthoxycarbonylamino)-éthyl]-phosphinoyl}-propionylamino)-propionique

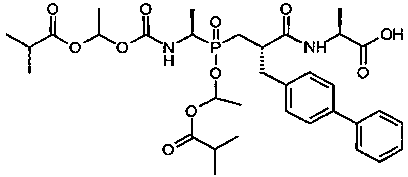

### Etape 1: Ester 1-{1-[[2-(1-benzyloxycarbonyl-éthylcarbamoyl)-3-biphényl-4-yl-propyl]-(1-isobutyryloxy-éthoxy)-phosphinoyl]-éthylcarbamoyloxy}-éthyl de l'acide isobutyrique

Le composé final de l'exemple 3 (350mg) est mis en solution dans le toluène. On ajoute sous atmosphère inerte 90 mg de sulfate de tetrabutylammonium, (nBu)₄N⁺SO₄H⁻ (0,5 éq.), NaI (80mg, 1éq.), l'ester 1-chloroethylique de l'acide isobutyrique (160mg, 4éq.) et 0,9 mL de DIEA. Le mélange est chauffé à 120°C pendant 3h. Le mélange réactionnel est ramené à température ambiante et est dilué dans 50 mL d'acétate d'éthyle, la solution obtenue est lavée à l'eau, avec une solution aqueuse de NaHCO₃ à 10%, avec une solution aqueuse saturée de NaCl et séchée sur Na₂SO₄. Après filtration, les solvants sont évaporés et le résidu (400 mg) est purifié par chromatographie sur gel de silice avec le mélange CH₂Cl₂/MeOH 90/10 comme éluant. Huile jaune, 200mg.
CLHP (CH₃CN (0,1 % TFA)/ H₂O (0,1% TFA) 80/20, Colonne Kromasil C18) Rt=8,8 mn.
Masse ESI(+) [M+H]⁺=781.

### Etape 2 : Acide 2-(2-biphényl-4-ylméthyl-3-{(1-isobutyryloxy-éthoxy)-[1-(1-isobutyryloxy-éthoxycarbonylamino)-éthyl]-phosphinoyl}-propionylamino)-propionique

Le composé de l'étape 1 (200mg) est solubilisé dans MeOH (10mL). On ajoute 40 mg de Pd/C à 10% et le mélange est hydrogénolysé à température et pression ordinaire pendant 1h sous atmosphère d'hydrogène. Le mélange est dilué dans CH₂Cl₂ et filtré sur célite. Le solvant est évaporé à sec et le résidu est lyophilisé. Solide blanc, 106 mg (60%).
Masse ESI (+) [M+H]⁺ = 691.
RMN 1H (DMSOd6) δ (ppm) :1,0-1,5 (24H m); 1,5-1,7 (2H m); 2,5 (2H m); 2,7-3,0 (3H m); 3,9 (1H m); 4,2 (1H m); 6,4 (1H m); 6,65 (1H m); 7,2-7,7 (9H+1H m); 8,3 (1H m).

### Exemple 10 : Ester benzylique de l'acide 2-(4-bromo-benzyl)-acrylique

### Etape 1 Acide 2-(4-bromo-benzyl)-acrylique

Ce composé est synthétisé en suivant le protocole décrit pour la synthèse de l'acide 2-Biphenyl-4-ylmethyl-acrylique (exemple2) en remplaçant, le biphényl-4-yl-acetaldéhyde par le 4-bromo benzaldéhyde. Solide blanc.
CLHP (CH₃CN (0,1 % TFA)/ H₂O (0,1% TFA) 70/30, Colonne Kromasil C18) Rt= 4,9 mn.

### Etape 2 : Ester benzylique de l'acide 2-(4-bromo-phényl-4-ylméthyl-acrylique

L'estérification est effectuée en suivant le protocole décrit dans l'étape 3 de l'exemple 2. Huile incolore.
CLHP (CH₃CN (0,1 % TFA)/ H₂O (0,1% TFA) 70/30, Colonne Kromasil C18) Rt= 22,0 mn.
RMN (CDCl3) δ (ppm) : 3,6 (2Hs); 5,2 (2H s); 5,6 (1H d); 6,4 (1H d); 7,1 (2H d); 7,4 (7H m).

### Exemple 11 : Ester benzylique de l'acide 2-(2-(4-bromo-benzyl)-3-{hydroxy-[1-(1-isobutyryloxy-éthoxycarbonylamino)-éthyl]-phosphinoyl}-propionylamino)-propionique

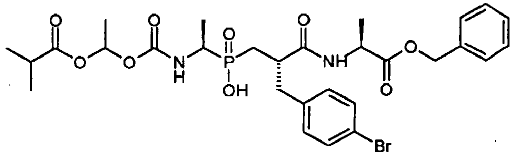

Ce composé est synthétisé en suivant le protocole décrit dans l'exemple 3 en remplaçant l'ester benzylique de l'acide 2-biphényl-4-ylméthyl-acrylique par l'ester benzylique de l'acide 2-(4-bromo-benzyl)-acrylique

### Etape 1 : Ester benzylique de l'acide 3-[(1-benzyloxycarbonylamino-éthyl)-hydroxy-phosphinoyl]-2-(4-bromo-benzyl)-propionique

Le composé de l'exemple 1 (5,15 g) de configuration R et le composé de l'exemple 10 (7,21 g) sont mis en solution dans le BSA (18 mL) et le mélange est chauffé pendant 30h à 75°C. Le mélange réactionnel est ramené à température ambiante, et est dilué dans l'acétate d'éthyle. La phase organique est lavée à l'eau puis évaporée à sec. On obtient un produit solide blanc correspondant au mélange des deux diastéréoisomères *1R,2S et 1R*/*2R* dans des proportions relatives 65/35. Par recristallisation dans l'acétonitrile on obtient l'isomère 1R,2S avec un excès énantiomérique ee > 97%. Solide blanc, 3,35g (42%).
RMN (DMSOd6) δ (ppm) : 1,2 (3H dd); 1,7-2,2 (2H m); 2,7-3,1 (3H m); 3,8 (1H p); 4,95 (2H dd); 5,05 (2H s); 7,0-7,4 14H m); 7,55 (1H m).

### Etape 2: Acide 3-((1-aminoéthyl)-hydroxy-phophinoyl)-2-(4-bromo-benzyl)-propionique

A partir de 3,3 g du composé de l'étape 1 et en suivant le protocole décrit dans l'étape 2 de l'exemple 3, on obtient le composé attendu sous forme d'un solide blanc, 2,5 g (98%).

### Etape 3 : Acide 2-(4-bromo-benzyl)-3-{hydroxy-[1-(1-isobutyryloxy-éthoxycarbonylamino)-éthyl]-phosphinoyl}-propionique

A partir de 2,5 g du composé de l'étape 2 et en suivant le protocole décrit dans l'étape 3 de l'exemple 3, on obtient le composé attendu. Solide blanc, 2,6g.
Masse (M+H)⁺=508-510
RMN (DMSOd6) δ (ppm) : 1,0-1,5 (12 Hm); 1,7 (2H m); 2,5 (1H m); 2,9 (3H m); 3,8 (1H)m; 6,6 (1H m); 7,1 (2H d); 7,4 (2H d); 7,75 (1H m).

### Etape 4: Ester benzylique de l'acide 2-(2-(4-bromo-benzyl)-3-{hydroxy-[1-(1-isobutyryloxy-éthoxycarbonylamino)-éthyl]-phosphinoyl}-propionylamino)-propionique

A partir d'1 g du composé de l'étape 3 et 388 mg d'ester benzylique de l'alanine, on obtient en suivant le protocole de l'étape 4 de l'exemple 3, le composé attendu. Solide blanc.
Masse (M+H)⁺=669-671
RMN(DMSOd6) δ (ppm) :1,0-1,3 (6H d); 1,25 (6H m); 1,45 (3H d); 1,6-1,9 (2H m) ; 2,5 (1H m); 2,7-3,0 (3H m); 3,7 (1H m); 4,3 (1H m); 5,0 2H dd)); 6,6 (1H m); 7,10 (2H d); 7,3 (4H s, +2H d); 7,5 (1H m), 8,4 (1H dd).

### Exemple 12 : Acide 2-(2-(4-bromo-benzyl)-3-{hydroxy-[1-(1-isobutyryloxy-éthoxycarbonylamino)-éthyl]-phosphinoyl}-propionylamino)-propionique

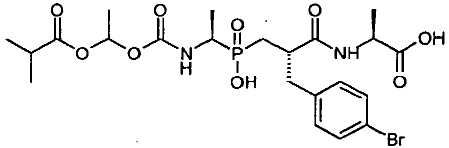

### Etape 1 : Acide 3-((1-benzyloxycarbonylamino-éthyl)-hydroxy-phosphinoyl)-2-(4-bromo-benzyl)-propionique.

Le composé de l'étape 1 de l'exemple 11 (1,2g) est solubilisé dans le THF (10 mL) et 0,7 g de NaOH en solution dans 5 mL d'eau est ajouté. Le mélange est agité à température ambiante pendant 3h. Le THF est évaporé, la phase aqueuse est acidifiée à pH=1 par une solution d'HCl 1N, puis extraite par l'acétate d'éthyle. La phase organique est séchée sur Na₂SO₄, filtrée et évaporée à sec. Solide blanc, 0,9g (90%).

### Etape 2: Ester t-butylique de l'acide 2-(3-((1-benzyloxycarbonylamino-éthyl)-hydroxy-phosphinoyl)-2-(4-bromo-benzyl)-propionylamino)-propionique.

Le composé de l'étape 1 (0,9 g) est solubilisé dans 10 mL de DMF sous azote, puis on ajoute successivement le chlorhydrate de l'alaninate de t-butyle (450 mg), 2,5 mL de DIEA (6 eq) et 1,98 g de TBTU (3eq). Le mélange est agité 15 min à température ambiante, puis la réaction est traitée comme dans l'étape 4 de l'exemple 3. On obtient 1,10g du produit attendu (97%).

### Etape 3: Acide 2-(3-((1-amino-éthyl)-hydroxy-phosphinoyl)-2-(4-bromo-benzyl)-propionylamino)-propionique

Le composé obtenu dans l'étape 2 est solubilisé dans un mélange CH₂Cl₂/TFA 50/50 (40 mL) et le mélange est agité 1h à température ambiante. La solution est évaporée à sec, le résidu est repris dans l'eau et lyophilisé. On obtient 1,0 g du composé attendu.

Celui-ci est solubilisé dans un mélange HBr/AcOH (10 mL) et la solution est agitée 1h à température ambiante. Le mélange est ensuite évaporé sous pression réduite. Le résidu est repris dans l'eau et lyophilisé. On obtient 930 mg du produit attendu. Etape 4 : Acide 2-(2-(4-bromo-benzyl)-3-{hydroxy-[1-(1-isobutyryloxy-éthoxycarbonylamino)-éthyl]-phosphinoyl}-propionylamino)-propionique

Le composé précédent est solubilisé dans 15 mL de CH₂Cl₂. On ajoute 0,76 g de isobutyloxyethyl succinimidyl carbonate et 1,87 mL de DIEA et le mélange est agité 1 h à température ambiante. Le mélange est ensuite lavé par H₂O et par une solution aqueuse saturée de NaCl, séché sur Na₂SO₄ et évaporé à sec. Le produit brut est purifié par CLHP.
CLHP (colonne C18 ACE, solvant CH₃CN(0,1% TFA) /H₂O (0,1%TFA) 40 /60) Rt 8,66 mn.
ESI : (M+H)+= 578-580
RMN (DMSO d6) δ (ppm) : 1,0 (6H d); 1,1 (3H m); 1,2 (3H d); 1,35 (3H d); 1,6-1,9 (2H m); 2,5 (1H m); 2,7-2,9 (3H m); 3,7 (1H m); 4,1 (1H m); 6,6 (1H m); 7,1 (2H d); 7,4 (2H,d); 7,6 (1H dd); 8,2 (1H dd).

### Exemple 13 : Acide (R)-(benzyloxycarbonylamino)(thiophen-3-yl)methyl-phosphinique

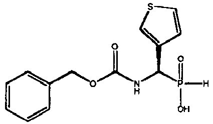

### Etape 1 : Acide (benzhydrylamino)(thiophen-3-yl)methylphosphinique

Un mélange de 20,0g (80 mmole) d'ortho phosphate de diphénylméthylamine dans 60 mL d'éthanol anhydre est porté au reflux sous agitation. On ajoute 160,0 mmole de 3-Thiophène carboxaldéhyde en solution dans 19 mL d'éthanol, goutte à goutte en 30 mn.

La réaction est suivie par CLHP. Après 2,5 h, la réaction est ramenée à température ambiante. Un mélange Et₂O/Acétone (60 mL) est ajouté au mélange. Le précipité blanc obtenu est filtré, lavé à l'eau (2x50 mL) et à l'acétone (2x50 mL) puis séché. Solide blanc, 15,4 g (56%).

### Etape 2 : Acide amino(thiophen-3-yl)methylphosphinique

Un mélange de 15 g du composé de l'étape 1 et de 122 mL d'HCl 6N est porté au reflux pendant 2h. Après refroidissement, le milieu réactionnel est concentré de moitié puis extrait par 3x1,5 L d'éther éthylique. La solution est évaporée à sec et le résidu huileux est repris par 120 mL d'éthanol. La solution est refroidie à 0°C et 30 mL d'oxyde de propylène sont ajoutés. Un solide blanc précipite. Le précipité est essoré, lavé à l'éthanol (2x20 mL), à l'éther éthylique (2x10 mL) et séché. Solide blanc, 7,32 g (95,1%).
RMN (1H) DMSO d6, δ (ppm): 4,92 (1H dd); 6,77 (1H, d); 7,10-7,50 (3H, m); 8,23 (3H m).

### Etape 3 : Acide (benzyloxycarbonylamino)(thiophen-3-yl)methylphosphinique

Le composé de l'étape 2 est mis en suspension dans 25 mL de dioxanne. Le pH est ajusté à 9,5 par ajout de soude (23 mL). 6,46 mL de chloroformiate de benzyle sont ajoutés sous agitation. Après 3h à 0°C, le mélange est ramené à température ambiante et est versé sur un mélange de glace (IL) et d'HCl concentré (30 mL). Le précipité blanc formé est essoré, lavé à l'eau (2x20 mL) et est séché. Solide blanc, 7,09 g (47%).
CLHP (CH₃CN (0,1 % TFA)/ H₂O (0,1% TFA) 70/30, Colonne Kromasil C18), Rt : 12,19 mn.

La résolution de l'acide phosphinique racémique est effectuée par recristallisation du sel obtenu avec la (R) (+) α-methylbenzylamine, comme décrit dans Baylis et al. (J. Chem. Soc. Perkin Trans I 1984, 2845) dans un mélange acétate d'éthyle/isopropanol=3,5/1. Solide blanc,1,42g (27%).
RMN (1H) DMSO d6 δ (ppm): 4,92 (1H dd); 5,05 (2H, s); 6,77 (1H, d); 7,10-7,50 (8H, m); 8,20 (1H, d).

### Exemple 14 : Acide (R)-(benzyloxycarbonylamino)(phényl)methylphosphinique

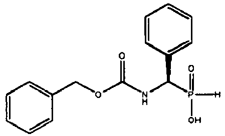

### Etape 1 : Acide (benzhydrylamino)(phényl)methylphosphinique

Un mélange de 24,95 g (100 mmole) de ortho-phosphate de diphénylméthylamine dans 75 mL d'éthanol anhydre est porté au reflux sous agitation. On ajoute 200 mmole de benzaldéhyde en solution dans 24 mL d'éthanol, goutte à goutte en 30 mn. La réaction est suivie par CLHP. Après 2,5 h, la réaction est ramenée à température ambiante. Un mélange Et₂O/Acétone (75 mL) est ajouté au mélange. Le précipité blanc obtenu est filtré, lavé à l'eau (2x60 mL) et à l'acétone (2x60 mL) puis séché. Solide blanc, 20,2g (60,1 %).

### Etape 2 : Acide amino(phényl)methylphosphinique

Un mélange de 20g du composé de l'étape 1 et de 160 mL d'HCl 6N est porté au reflux pendant 2h. Après refroidissement, le milieu réactionnel est concentré de moitié puis extrait par 3x1,5 L d'éther éthylique. La solution est évaporée à sec et le résidu huileux est repris par 160 mL d'éthanol. La solution est refroidie à 0°C et on ajoute 40 mL d'oxyde de propylène. Un solide blanc précipite. Le précipité est essoré, lavé à l'éthanol (2x20 mL), à l'éther éthylique (2x10 mL) et séché. Solide blanc, 7,25g (95,3 %).
RMN (1H) DMSO d6 δ (ppm): 4,92 (1H dd); 6,77 (1H, d); 7,10-7,50 (5H, m); 8,23 (3H, m).

### Etape 3 : Acide (benzyloxycarbonylamino) (phényl))methylphosphinique

Le composé de l'étape 2 est mis en suspension dans 25 mL de dioxanne. Le pH est ajusté à 9,5 par ajout de soude (23 mL). On ajoute sous agitation 6,46 mL de chloroformiate de benzyle. Après 3h à 0°C, le mélange est ramené à température ambiante et est versé sur un mélange de glace (IL) et d'HCl concentré (30 mL). Le précipité blanc formé est essoré, lavé à l'eau (2x20 mL) et séché. Solide blanc, 11,44g (88,5%).
CLHP (CH₃CN (0,1 % TFA)/H₂0 (0,1% TFA) 40/60, Colonne Kromasil C18), Rt 3,90 min.
La résolution de l'acide phosphinique racémique est effectuée par recristallisation du sel obtenu avec la (R) (+) α-methylbenzylamine, comme décrit dans Baylis et al. (J. Chem. Soc. Perkin Trans I 1984, 2845) dans un mélange acétate d'éthyle/isopropanol=3,5/1. Solide blanc, 3,9g (34,0%)
RMN (1H) CDCl₃ δ (ppm) : 4,92 (1H dd); 5,0 (2H, s); 6,77 (1H, d); 7,10-7,50 (10H, m); 8,31 (1H, d).

### Exemple 15 : Ester methylique de l'acide 2-(4-Thiophen-3-yl-benzyl)-acrylique

Ce composé est synthétisé en suivant le protocole décrit pour la synthèse de l'acide 2-Biphenyl-4-ylmethyl-acrylique (exemple 2) en remplaçant, le biphényl-4-yl-acetaldéhyde par le 4-bromo benzaldéhyde. Solide blanc (48,0%).

CLHP (CH₃CN (0,1 % TFA)/H₂O (0,1% TFA) 70/30, Colonne Kromasil C18) Rt= 11,28 min.

### Exemple 16: Acide 2-[2-{Hydroxy-[(1-isobutyryloxy-ethoxycarbonylamino)-phenyl-methyl]-phosphinoylmethyl}-3-(4-thiophen-3-yl-phenyl)-propionylamino]-propionique

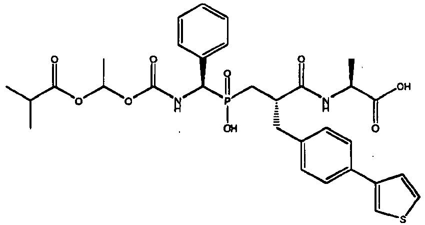

Ce composé est synthétisé en suivant le protocole décrit dans l'exemple 3 en remplaçant l'ester benzylique de l'acide 2-biphényl-4-ylméthyl-acrylique par l'ester méthylique de l'acide 2-(4-thiophen-3-yl-benzyl)-acrylique (exemple 15).

### Etape 1 : Ester éthylique de l'acide 3-[(1-benzyloxycarbonylamino-éthyl)-hydroxy-phosphinoyl]-2-(4-Thiophen-3-yl-benzyl)-propionique

Le composé de l'exemple 14 (15,42 g ; 1,2 éq) de configuration R et le composé de l'exemple 15 (13,82 g; 1,0 éq) sont mis en solution dans le BSA (50 mL) et le mélange est chauffé pendant 15h à 75°C. Le mélange réactionnel est ramené à température ambiante, et est dilué dans l'acétate d'éthyle. La phase organique est lavée à l'eau puis évaporée à sec. On obtient un produit solide blanc correspondant au mélange des deux diastéréoisomères *1R,2S et 1R,2R* dans des proportions relatives 65/35. Solide blanc, 17,26g (68%).
CLHP (CH₃CN (0,1 % TFA)/H₂O (0,1% TFA) 70/30, Colonne Kromasil C18) Rt= 6,51 min.
RMN (DMSOd6) δ (ppm) 1,50 (3H, t), 1,81-2,16 (2H, m); 2,78-3,12; (2H, m), 3,68 (3H, s); 3,78 (1H, qt); 4,50 (2H, q); 4,90 (1H, m); 5,20 (2H, s); 7,12-8,15 (17 H arom. +NH , m).

### Etape 2 : Bromhydrate de l'ester éthylique de l'acide (R)-amino(phenyl)methyl-3-ethoxy-3-oxo-2-(4-(thiophen-3-yl)benzyl)propyl)phosphinique

17,86 g du composé de l'étape 1 est solubilisé dans 180 ml de HBr 48% dans AcOH. Le mélange est agité 1h à température ambiante et le mélange est évaporé sous pression réduite pour donner le produit à l'état brut sous forme d'huile (100%) RMN (DMSOd6) δ (ppm) 1,50 (3H, t); 1,81-2,16 (2H, m); 2,78-3,12, (2H, m); 3,68 (3H, s); 3,78 (1H, qt); 4,50 (2H, q); 4,90 (1H, m); 5,20 (2H, s); 7,12-8,0 (12 H, m); 8,15 (3H, m).

### Etape 3 : Ester éthylique de l'acide (R)-(tert-butoxycarbonylamino) (phenyl)methyl((S)-3-ethoxy-3-oxo-2-(4-(thiophen-3-yl)benzyl)propyl)phosphinique

Le composé de l'étape 2 (30,9mmol) est solubilisé dans 300mL de DMF. A cette solution, sont ajoutés Et₃N (35 mL, 250 mmol, 8 eq) et Boc₂O (6.75 g, 1eq) dans 50 mL de DMF. Le mélange est agité la nuit à température ambiante. Le DMF est évaporé sous pression réduite et le mélange est repris par AcOEt. La phase organique est lavée par une solution d'acide citrique 10%, une solution de NaHCO₃ 10%, une solution de NaCl sat., séchée sur Na₂SO₄ et concentrée sous pression réduite pour donner 15.16g de produit (90,2%).
CLHP (CH₃CN (0,1 % TFA)/H₂O (0,1% TFA) 70/30, Colonne Kromasil C18) Rt= 5,42 min.
RMN (DMSOd6) δ (ppm) 1,50 (3H, t); 1,45 (9H, s); 1,81-2,16 (2H, m); 2,78-3,12, (2H, m); 3,78 (1H, qt); 4,50 (2H, q); 4,90 (1H, m); 7,12-8,0 (12 H aromatique + NH, m).

### Etape 4: Acide 3-(((R)-(tert-butoxycarbonylamino)(phenyl)methyl)(hydroxy) phosphoryl)-2-(4-(thiophen-3-yl)benzyl)propanoique

Le composé de l'étape 3 (15,16g ; 27,88 mmol) est solubilisé dans 280 ml d'acétone puis NaOH 1N (278,8 mL, 10 eq) sont ajoutés goutte à goutte. Le mélange est agité la nuit à température ambiante puis l'acétone est évaporée sous pression réduite. Le mélange est repris par AcOEt. La phase aqueuse est extraite puis est acidifiée par HCl 1N. La phase aqueuse est alors extraite par AcOEt. La phase organique est ensuite lavée par H₂0, NaCl sat., séchée sur Na₂SO₄ et évaporée sous pression réduite pour donner 13,73g d'huile (95%).
CLHP (CH₃CN (0,1 % TFA)/H₂O (0,1% TFA) 50/50, Colonne Kromasil C18) Rt= 11,40 min.
RMN (DMSOd6) δ (ppm) 1,45 (9H, s); 1,81-2,16 (2H, m); 2,78-3,12, (2H, m); 3,78 (1H, qt); 4,90 (1H, m); 7,12-8,0 (12 H aromatique + NH, m).

### Etape 5 : Ester méthylique de l'acide (R)-(tert-butoxycarbonylamino)(phenyl) methyl((S)-3-((S)-1-methoxy-1-oxopropan-2-ylamino)-3-oxo-2-(4-(thiophen-3-yl)benzyl)propyl)phosphinique

A partir de 150 mg du composé de l'étape 4 et 52 mg d'ester méthylique de l'alanine, on obtient en suivant le protocole de l'étape 4 de l'exemple 3, le composé attendu. Le diastéréoisomère attendu est obtenu par purification par CLHP semi préparative sur colonne Kromasil C18 avec CH₃CN (0,1% TFA)/H₂0 (0,1 % TFA) 50/50 comme système d'élution. Solide blanc : 50 mg (30,0%).
CLHP (CH₃CN (0,1 % TFA)/H₂O (0,1% TFA) 70/30, Colonne Kromasil C18) Rt= 6,0 min.
RMN (DMSOd6) δ (ppm): 1,10-1,50 (12H, m); 1,81-2,16 (2H, m); 2,78-3,12, (3H, m); 3,48 (3H, s); 3,78 (1H, qt); 4,25 (1H, d); 4,90 (1H, m); 7,12-7,81 (12 H + NH, m); 8.5 (1H, d).

### Etape 6 : Acide (R)-(tert-butoxycarbonylamino)(phenyl)methyl((S)-3-((S)-1-methoxy-1-oxopropan-2-ylamino)-3-oxo-2-(4-(thiophen-3-yl)benzyl)propyl) phosphinique

50 mg du composé de l'étape 5 est mis en solution dans 2 mL d'acétone. 800 µL de NaOH 1N (10 éq) sont ajoutés et le mélange est agité 2,5 h à température ambiante puis l'acétone est évaporée sous pression réduite. Le mélange est repris par AcOEt. La phase aqueuse est extraite puis est acidifiée par HCl 1N. La phase aqueuse est alors extraite par AcOEt. La phase organique est ensuite lavée par H₂O, NaCl sat., séchée sur Na₂SO₄ et évaporée sous pression réduite pour donner 48 mg (98%). CLHP (CH₃CN (0,1 % TFA)/H₂O (0,1% TFA) 50/50, Colonne Kromasil C18) Rt= 7,91 min.
RMN (DMSOd6) δ (ppm) : 1,10-1,50 (12H, m); 1,81-2,16 (2H, m); 2,78-3,12, (3H, m); 3,78 (1H, qt); 4,25 (1H, d); 4,90 (1H, m); 7,12-7,81 (12 H +NH, m); 8.5 (1H, d).

### Etape 7: Trifluoroacétate de l'acide (2S)-2-((2S)-3-(((R)-amino(phenyl)methyl) (hydroxy)phosphoryl)-2-(4-(thiophen-3-yl)benzyl)propanamido)propanoique

48 mg du composé de l'étape 6 est mis en solution dans 4 mL de DCM. 60µL d'acide trifluoroacétique sont ajoutés et le mélange est agité 45 min à 0°C. Le mélange est évaporé sous pression réduite pour donner 49 mg (100%).
CLHP (CH₃CN (0,1 % TFA)/H₂O (0,1% TFA) 50/50, Colonne Kromasil C18) Rt= 2,60 min.
RMN (DMSOd6) δ (ppm) : 1,20 (3H, d); 1,81-2,16 (2H, m); 2,78-3,12, (3H, m); 3,78 1H (qt.); 4,25 (1H, d); 4,90 (1H, m); 7,12-7,81 (12H, m); 8,4 (1H, d); 8,7 (3H, m).

### Etape 8: Acide 2-[2-{Hydroxy-[(1-isobutyryloxy-ethoxycarbonylamino)-phenyl-methyl]-phosphinoylmethyl}-3-(4-thiophen-3-yl-phenyl)-propionylamino]-propionique

Le composé précédent est solubilisé dans 1 mL de CH₃CN puis 340µL de NaHCO₃ 2N.et 34 mg de isobutyloxyethyl succinimide carbonate (1.2 éq.) sont ajoutés le mélange est agité 1 h à 60°C. Le mélange est repris par de l'acétate d'éthyle puis est lavé par H₂O et par une solution aqueuse saturée de NaCl, séché sur Na₂SO₄ et évaporé à sec. Le produit brut est purifié par CLHP.
CLHP (CH₃CN (0,1 % TFA)/H₂O (0,1% TFA) 38/62, Colonne Atlantis T3) Rt= 10,52 min.
RMN (DMSOd6) δ (ppm) : 1,0-1,3 (6H d); 1,20 (3H, d); 1,25 (3H, d); 1,81-2,16 (2H, m); 2,78-3,12, (3H, m); 3,78 1H (qt.); 4,25 (1H, d); 4,90 (1H, m); 6,64 (1H m); 7,12-7,81 (13H, m); 8,28 (1H, d).

### Exemple 17: Acide 2-[2-{Hydroxy-[(1-isobutyryloxy-ethoxycarbonylamino)-phenyl-methyl]-phosphinoylmethyl}-3-(4-thiophen-3-yl-phenyl)-propionylamino]-3-hydroxypropionique

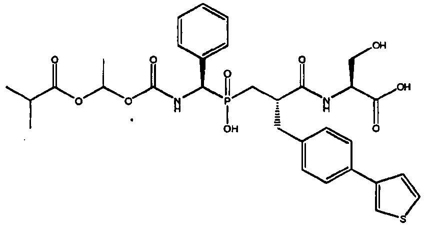

Ce composé est synthétisé en suivant le protocole décrit dans l'exemple 16en remplaçant l'ester méthylique de l'alanine par l'ester méthylique de la serine (O-tBu).
CLHP (CH₃CN (0,1 % TFA)/H₂O (0,1% TFA) 38/62, Colonne Atlantis T3) Rt= 10,02 min.
RMN (DMSOd6) δ (ppm) : 1,0-1,3 (6H, d); 1,20 (3H, d); 1,81-2,16 (2H, m); 2,78-3,12, (3H, m); 3,40-3,60 (2H, m); 3,78 (1H, qt); 4,25 (1H, d); 4,90 (1H, m); 6,64 (1H m); 7,12-7,81 (13H, m); 8,28 (1H, d).

### Exemple 18 : Acide 2-(3-Biphenyl-4-yl-2-{hydroxy-[(1-isobutyryloxy-ethoxycarbonylamino)-thiophen-3-yl-methyl]-phosphinoylmethyl}-propionylamino)-propionique

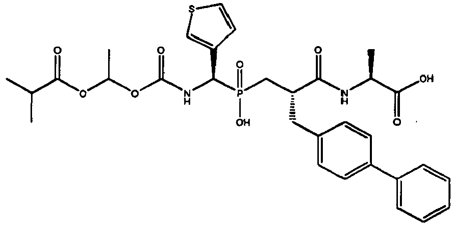

### Etape 1 : 3-(((R)-(benzyloxycarbonylamino)(thiophen-3-yl)methyl)(hydroxy) phosphoryl)-2-(biphenyl-4-ylmethyl)propanoique

Le composé de l'exemple **13** (660 mg ; 1,0 éq) de configuration *R* et le composé de l'exemple **2,** étape 2 (642 mg ; 1,2 éq) sont mis en solution dans le BSA (5 mL) et le mélange est chauffé pendant 15h à 75°C. Le mélange réactionnel est ramené à température ambiante, et est dilué dans l'acétate d'éthyle. La phase organique est lavée à l'eau puis évaporée à sec. On obtient un produit solide blanc correspondant au mélange des deux diastéréoisomères de configuration respective *1R*,*2S et 1R,2R* dans des proportions relatives 65/35. Solide blanc, 1,4g (100%).
RMN (DMSOd6) δ (ppm): 1,60-2,00 (2H, m); 2,78-3,12, (2H, m); 3,78 (1H, qt); 4,90 (1H, m); 5,20 (2H, s); 7,12-8,15 (17 H arom. +NH , m).

### Etape 2 : Acide (R)-(benzyloxycarbonylamino)(thiophen-3-yl)methyl(2-(biphenyl-4-ylmethyl)-3-((S)-1-methoxy-1-oxopropan-2-ylamino)-3-oxopropyl)phosphinique

A partir de 2,12 mmol du composé de l'étape 1 et 2,75 mmol d'ester méthylique de l'alanine, on obtient en suivant le protocole de l'étape 4 de l'exemple 3, le composé attendu. Solide blanc: 1,40g (90,0%).
RMN (DMSOd6) δ (ppm): 1,25-1,40 (3H, m); 1,60-2,00 (2H, m); 2,78-3,12, (2H, m); 3,40 (3H, s); 3,78 (1H, qt); 4,25 (1H, m); 4,90 (1H, m); 5,20 (2H, s); 7,12-8,15 (17 H arom. +NH , m).

### Etape 3: Acide 2-{2-[(Benzyloxycarbonylamino-thiophen-3-yl-methyl)-hydroxy-phosphinoylmethyl]-3-biphenyl-4-yl-propionylamino}-propionique

100 mg du composé de l'étape 2 est mis en solution dans 4 mL d'acétone. 1,6 mL de NaOH 1N (10 éq) sont ajoutés et le mélange est agité pendant 2,5 h à température ambiante puis l'acétone est évaporée sous pression réduite. Le mélange est repris par AcOEt. La phase aqueuse est extraite puis est acidifiée par HCl 1N. La phase aqueuse est alors extraite par AcOEt. La phase organique est ensuite lavée par H₂O, NaCl sat., séchée sur Na₂SO₄ et évaporée sous pression réduite pour donner 98mg (98%).
RMN (DMSOd6) δ (ppm): 1,25-1,40 (3H, m); 1,60-2,00 (2H, m); 2,78-3,12, (2H, m); 3,78 (1H, qt); 4,25 (1H, m); 4,90 (1H, m); 5,20 (2H, s); 7,12-8,15 (17 H arom. +NH , m).

### Etape 4: Trifluoroacétate de l'acide (2S)-2-((2S)-3-(((R)-amino(thiophen-3-yl)methyl)(hydroxy)phosphoryl)-2-(biphenyl-4-ylmethyl)propanamido)propanoique

Le composé de l'étape 3 est mis en solution dans 4 mL de HBr 48% en solution dans l'acide acétique. Le mélange est agité pendant 2h à température ambiante. Le mélange est évaporé sous pression réduite et le résidu est purifié par CLHP semi préparative sur colonne Kromasil C18 avec CH₃CN (0,1% TFA)/H₂O (0,1 % TFA) 50/50 comme système élution. Solide blanc : 28 mg (57,1%).
RMN (DMSOd6) δ (ppm): 1,25-1,40 (3H, m); 1,60-2,00 (2H, m); 2,78-3,12, (2H, m); 3,78 (1H, qt); 4,25 (1H, m); 4,90 (1H, m); 7,12-8,15 (12 H, m); 8.50 (3H, m).

### Etape 5 : Acide 2-(3-Biphenyl-4-yl-2-{hydroxy-[(1-isobutyryloxy-ethoxycarbonylamino)-thiophen-3-yl-methyl]-phosphinoylmethyl}-propionylamino)-propionique

Le composé précédent est solubilisé dans 1 mL de CH₃CN et 195µL de NaHCO₃ 2N, puis 20 mg de isobutyloxyethyl succinimide carbonate (1.2 éq.) sont ajoutés. Le mélange est agité pendant 1 h à 60°C. Le mélange est repris par de l'acétate d'éthyle ensuite lavé par H₂O et par une solution aqueuse saturée de NaCl, séché sur Na₂SO₄ et évaporé à sec. Le produit brut est purifié par CLHP.
CLHP (CH₃CN (0,1 % TFA)/H₂O (0,1% TFA) 38/62, Colonne Atlantis T3) Rt= 10,42 min.
RMN (DMSOd6) δ (ppm) : 1,0-1,3 (6H d); 1,20 (3H, d); 1,25 (3H, d); 1,80-2,20 (2H, m); 2,80-3,20, (3H, m); 3,75 1H (qt.); 4,25 (1H, d); 4,90 (1H, m); 6,65 (1H m), 7,10-7,80 (13H, m); 8,28 (1H, d).

### Exemple 19: Acide 2-{3-Biphenyl-4-yl-2-[hydroxy-(1-isobutyryloxymethoxy carbonylamino-ethyl)-phosphinoylmethyl]-propionylamino}-propionique

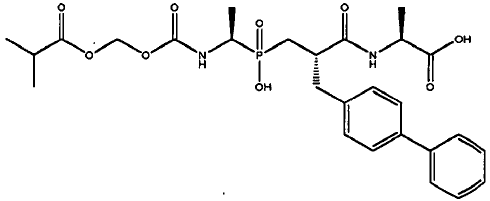

### Etape 1 : Acide 2-[(1-Benzyloxycarbonylamino-ethyl)-hydroxy-phosphinoylmethyl]-3-biphenyl-4-yl-propionique

Le composé de l'étape 1 de l'exemple 3 (1,2g) est solubilisé dans le THF (10 mL) et 0,7 g de NaOH en solution dans 5 mL d'eau est ajouté. Le mélange est agité à température ambiante pendant 3h. Le THF est évaporé, la phase aqueuse est acidifiée à pH=1 par une solution d'HCl 1N, puis extraite par l'acétate d'éthyle. La phase organique est séchée sur Na₂SO₄, filtrée et évaporée à sec. Solide blanc, 0,9g (90%). CLHP (CH₃CN (0,1 % TFA)/H₂O (0,1 % TFA) 38/62, Colonne ACE C18) Rt=17,16 min.
RMN 1H (DMSOd6): δ (ppm) 1,26 (3H, dd); 1,81-2,16 (2H, m); 2,78-3,12(3H, m); 3,78 (1H, qt); 5,20 (2H, q); 7,12-7,68 (14H arom. +NH (m).)

### Etape 2 : Ester t-butylique de l'acide 2-{2-[(1-Benzyloxycarbonylamino-ethyl)-hydroxy-phosphinoylmethyl]-3-biphenyl-4-yl-propionylamino}-propionique

Le composé de l'étape 1 (0,9 g) est solubilisé dans 10 mL de DMF sous azote, puis on ajoute successivement le chlorhydrate de l'alaninate de t-butyle (450 mg), 2,5 mL de DIEA (6 eq) et 1,98 g de TBTU (3eq). Le mélange est agité pendant 15 min à température ambiante, puis la réaction est traitée comme dans l'étape 4 de l'exemple 3. On obtient 1,10g du produit attendu (97%).
CLHP (CH₃CN (0,1 % TFA)/H₂O (0,1% TFA) 50/50, Colonne ACE C18) Rt= 11,93 min.
RMN 1H (DMSOd6): δ (ppm) 1,15-1,35 (6H, m); 1,42 (9H, s);1,81-2,16 (2H, m); 2,78-3,12(3H, m); 3,78 (1H, qt); 4,22 (1H, q); 5,20 (2H, q); 7,12-7,68 (14H arom. +NH (m).

### Etape 3: Acide 2 (2S)-2-((2S)-3-(((R)-1-aminoethyl)(hydroxy)phosphoryl)-2-(biphenyl-4-ylmethyl)propanamido)propanoique

Le composé obtenu dans l'étape 2 est solubilisé dans un mélange HBr/AcOH (10 mL) et la solution est agitée pendant 1h à température ambiante. Le mélange est ensuite évaporé sous pression réduite. Le résidu est repris dans l'eau et lyophilisé. On obtient 930 mg du produit attendu (100%).
CLHP (CH₃CN (0,1 % TFA)/H₂O (0,1% TFA) 50/50, Colonne ACE C18) Rt= 11,93 min.
RMN 1H (DMSOd6): δ (ppm) 1,15-1,35 (6H, m), 1,42 (9H, s),1,81-2,16 (2H, m), 2,78-3,12(3H, m), 3,78 (1H, qt), 4,22 (1H, q), 5,20 (2H , q), 7,12-7,68 (14H arom. +NH (m).

### Etape 4: Acide 2-{3-Biphenyl-4-yl-2-[hydroxy-(1-isobutyryloxymethoxy carbonylamino-ethyl)-phosphinoylmethyl]-propionylamino}-propionique

300 mg du composé de l'étape 3 est solubilisé dans 2 mL de CH₃CN et 2mL de NaHCO₃ 2N, puis, 186 mg de isobutyloxy succinimide carbonate (1.2 éq.) sont ajoutés. Le mélange est agité pendant 1 h à 60°C. Le mélange est repris par de l'acétate d'éthyle et est lavé par H₂O et par une solution aqueuse saturée de NaCl, séché sur Na₂SO₄ et évaporé à sec. Le produit brut est purifié par CLHP pour donner 170 mg du produit désiré (50.5%).
CLHP (CH₃CN (0,1 % TFA)/H₂O (0,1% TFA) 38/62, Colonne Atlantis T3) Rt= 8,15 min.
RMN(DMSOd6) δ (ppm) : 1,0-1,3 (6H, d); 1,25 (3H, dd); 1,45 (3H, d); 1,6-1,9 (2H, m); 2,5 (1H, m); 2,7-3,0 (3H, m); 3,7 (1H, m); 4,3 (1H, m); 5,65 (2H, s); 7,10-7,80 (9H, m); 8,4 (1H dd).

### Exemple 20: Acide 1-(1-{[3-biphenyl-4-yl-2-(1-carboxy-ethylcarbamoyl)-propyl]-hydroxy-phosphinoyl}-ethylcarbamoyloxy)-ethyl de l'acide 2-Dimethyl-propionique

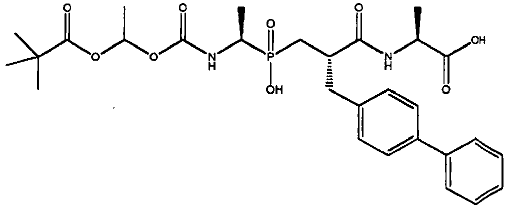

394 mg du composé de l'étape 3, exemple 19 sont solubilisés dans 2 mL de CH₃CN et 2mL de NaHCO₃ 2N, puis, 272 mg de isobutyloxy succinimide carbonate (1.2 éq.) sont ajoutés et le mélange est agité pendant 1 h à 60°C. Le mélange est repris par de l'acétate d'éthyle, lavé par H₂O et par une solution aqueuse saturée de NaCl, séché sur Na₂SO₄ et évaporé à sec. Le produit brut est purifié par CLHP pour donner 330 mg du produit désiré (71,6%).
CLHP (CH₃CN (0,1 % TFA)/H₂O (0,1% TFA) 38/62, Colonne Atlantis T3) Rt= 16,93 et 18,41 min.
RMN (DMSOd6) δ (ppm) : 1,20 (9H, d); 1,25 (3H, dd); 1,45 (3H, d); 1,6-1,9 (2H, m); 2,5 (1H, m); 2,7-3,0 (3H m); 3,7 (1H, m); 4,3 (1H, m); 6,6 (1H m); 7,10-7,80 (9H, m); 8,4 (1H dd).

### Exemple 21 : Acide 2-[(1-{Hydroxy-[1-(1-isobutyryloxy-ethoxycarbonylamino)-ethyl]-phosphinoylmethyl}-cyclopentanecarbonyl)-amino]-propionique

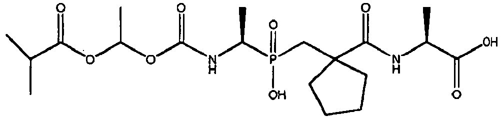

### Etape 1 : Ester t-butylique de l'acide cyclopentanoïque

L'acide cyclopentanoïque (15g, 0,131 mol), tBuOH (2,1 mL) et H₂SO₄ (750 µL) sont placés dans un flacon à parois épaisses. Environ 150 mL isobutylene condensé à - 78°C sont alors ajoutés. Le flacon est fermé, laissé revenir à température ambiante et agité 4 nuit à température ambiante.

Après évaporation de l'excès d'isobutylène, le mélange est repris dans Et₂O et lavé par NaHCO₃ 10%. La phase organique est séchée sur Na₂SO₄ et concentrée sous pression réduite pour donner 12.03g du produit attendu (Rdt : 80.6%).
RMN (CDCl3) δ (ppm): 1,40 (9H, d); 1,45-1.90 (8H, m); 2,55 (1H, m).

### Etape 2 : Ester t-butylique de l'acide 1-(hydroxymethyl)cyclopentanecarboxylique.

A une solution à 0°C de iPr₂NH (7.91 mL, 56.11 mmol) dans le THF (190mL) est additionné du nBuLi dans l'hexane (2.5 M, 23.3 mL, 58.17 mmol, 1.04 eq). Après 30 min, le mélange est refroidi à -78°C et une solution du composé de l'étape 1 (9.54g, 56.11 mmol) dans 25 mL de THF est ajoutée sous azote en 30 min. Le mélange est agité 1h à -78°C puis le paraformaldéhyde (5éq, 8.37g) est ajouté et le mélange est agité pendant 1h à -78°C puis pendant 12h à température ambiante.
190 mL d'une solution de NH₄Cl sat. sont ajoutés et le mélange est extrait par AcOEt (2x200mL). La phase organique est lavée par HCl 1N, NaCl sat., séchée sur Na₂SO₄ et concentrée sous pression réduite pour donner le produit désiré (Rdt : 60%).
RMN (CDCl₃) δ (ppm): 1,40 (9H, d); 1,45-1.90 (8H, m); 3,50 (2H, s).
CLHP Atlantis T3 4.6 x 100mm, 3µm, gradient de CH₃CN (0.1% TFA)/H₂O (0.1% TFA) 10 à 90% CH₃CN en 30 min, Rt : 12.43 min.

### Etape 3 : Benzyl (1R)-1-(methoxyhydrophosphoryl)ethylcarbamate

Sous azote, le composé de l'étape 2, exemple 3 (6g, 24.67 mmol) est solubilisé dans un mélange MeOH/Toluène. Une solution de triméthylsilyldiazométhane est ajoutée goutte à goutte jusqu'à ce que la coloration persiste (ce qui correspond à la fin du dégagement gazeux). Le mélange est agité 1h à température ambiante plus le toluène est évaporé sous pression réduite. Le mélange est extrait par AcOEt). La phase organique est lavée par NaHCO₃ 10%, NaCl sat., séchée sur Na₂SO₄ et concentrée sous pression réduite pour donner 5.75 g du produit désiré (Rdt: 90.7%).
RMN (DMSO d6) δ ppm: 1,20-1,35 (3H, m); 3.60 (2H, m); 3,70-4,10 (1H, m); 5.05 (2H, d); 6,90 (1H, m); 7,20-7,50 (5H, m); 7,75 (1H, m).
CLHP Atlantis T3 4.6 x-100mm, 3µm, CH₃CN (0.1% TFA)/H₂O (0.1% TFA) 30 à CH₃CN en 30 min, Rt : 4.30 et 4.48 min.

### Etape 4: Ester tert-butylique de l'acide 1-((trifluoromethylsulfonyloxy)methyl) cyclopentanoïque.

Un mélange de pyridine de 6.80 mL (84.2 mmol) et de 50 mL de dichlorométhane est refroidi à -78°C sous azote. Une solution d'anhydride triflique, 6.80g, (24.14 mmol) dans 7mL de CH₂Cl₂ est ajoutée à l'aide d'une ampoule à brome. Après 10 min, une solution de 3,4g du composé de l'étape 2 (17 mmol) dans 14mL de CH₂Cl₂ est ajoutée goutte à goutte. Le mélange est agité pendant 30 min à -78°C puis est ramené à température ambiante. 300mL d'hexane sont ajoutés. La phase organique est lavée par HCl 1N, H₂O, NaCl sat., séchée sur Na₂SO₄ et concentrée sous pression réduite pour donner 5.67 g du produit désiré (Rdt : 100%) utilisé tel quel pour l'étape suivante.
RMN (CDCl₃) δ (ppm): 1,40 (9H, d); 1,45-1.90 (8H, m); 4,65 (2H, s).

### Etape 5: Acide 1-((((R)-1-(benzyloxycarbonylamino)ethyl)(hydroxy)phosphoryl) methyl) cyclopentanecarboxylique.

Une solution d' iPr₂NH (2.19 mL, 15.64 mmol) dans THF (17mL) est refroidie à 0°C puis 6,23 mL de nBuLi 2.5 M dans l'hexane (15.64 mmol, 1.04 eq) sont ajoutés. Après 15 min, le mélange est refroidi à -78°C et une solution du composé de l'étape 3 (3.64g, 14.16 mmol) dans 28 mL de THF est ajouté sous azote de manière à maintenir la température inférieure à -60°C. Le mélange est agité 10 min à -78°C puis le composé de l'étape 4 (17 mmol) est ajouté dans 15 mL de THF et le mélange est agité pendant 15 min à -78°C puis pendant 4h à température ambiante.

Le mélange est dilué par de l'AcOEt. La phase organique est lavée par HCl 1N, NaHCO₃ 10%, NaCl sat., séchée sur Na₂SO₄ et concentrée sous pression réduite.

Le produit brut obtenu est mis en solution dans 10 mL de CH₂Cl₂ et 3.2mL de TFA sont ajoutés. Le mélange est agité 2h à 0°C. Après évaporation à sec, le produit attendu est purifié par CLHP semipréparative sur colonne ACE C18, CH₃CN (0.1% TFA)/ H₂0 (0.1 % TFA) 40/60 pour donner 120 mg du produit pur (Rdt : 29.4%). RMN (DMSO d6) δ (ppm): 1,20-1,35 (3H, m); 1,50-2,20 (10H, m); 3.75 (1H, q); 5.05 (2H, d); 5.05 (2H, d); 7,20-7,60 (6H, m).
CLHP ACE C18 4.6x250mm, 5µm, CH₃CN (0.1% TFA)/H₂O (0.1% TFA) 40/60, Rt : 5.57 min.

### Etape 6: Acide (R)-1-(benzyloxycarbonylamino)ethyl((1-((S)-1-tert-butoxy-1-oxopropan-2-ylcarbamoyl)cyclopentyl)methyl)phosphinique.

Le composé de l'étape 5 (0,118 g, 0.319 mmol) est solubilisé dans 2 mL de DMF sous azote, puis on ajoute successivement le chlorhydrate de l'alaninate de t-butyle (70 mg), 279µL de DIEA (5 eq) et 308 mg de TBTU (3eq). Le mélange est agité 15 min à température ambiante, puis la réaction est traitée comme dans l'étape 4 de l'exemple 3. On obtient 111 mg du produit attendu (70.3%).
CLHP (CH₃CN (0,1 % TFA)/ H₂O (0,1% TFA) 50/50, Colonne ACE (C18) Rt= 7.71 min.
RMN 1H (DMSOd6) δ (ppm): 1,15-1,35 (6H, m); 1,42 (9H, s); 1,40- 2,20 (10H, m); 3.85 (1H, q); 4,15 (1H, q); 5,05 (2H , s); 7,1-7,45 (5H, m); 7.90 (1H, d).

### Etape 7: Trifluoroacétate de l'acide (2S)-2-(1-((((R)-1-aminoethyl) (hydroxy)phosphoryl methyl)cyclopentanecarboxamido) propanoique

Le composé obtenu dans l'étape 6 est solubilisé dans un mélange HBr/AcOH (2 mL) et la solution est agitée 1h à température ambiante. Le mélange est ensuite évaporé sous pression réduite. Le produit est purifié par CLHP semi-préparative sur colonne ACE C18 avec un gradient de 0 à 60% de CH₃CN en 30 min d'un mélange CH₃CN (0.1%TFA))/ H₂O (0.1% TFA) pour donner 57.2 mg du produit attendu (Rdt : 61.9%). CLHP ACE C18 gradient de 0 à 60% de CH₃CN en 30 min d'un mélange CH₃CN (0.1 %)/ H₂0 (0.1% TFA) Rt: 9.97 min
RMN 1H (DMSOd6) δ (ppm): 1,15-1,35 (6H, m); 1,50-2,20 (10H, m); 3,40 (1H, q); 4,25 (1H, q); 7,85 (1H, d); 8,10 (3H , m).

### Etape 8 : Acide 2-[(1-{Hydroxy-[1-(1-isobutyryloxy-ethoxycarbonylamino)-ethyl]-phosphinoylmethyl} -cyclopentanecarbonyl)-amino]-propionic acid

55 mg du composé de l'étape 7 est solubilisé dans 1 mL de CH₃CN et 366 µL de NaHCO₃ 2N. 35 mg de isobutyloxy succinimide carbonate (1.2 éq.) sont ajoutés le mélange est agité 1 h à 60°C. Le mélange est ensuite repris par de l'acétate d'éthyle, lavé par H₂O et par une solution aqueuse saturée de NaCl, séché sur Na₂SO₄ et évaporé à sec. Le produit brut est purifié par CLHP pour donner 45 mg du produit attendu (71.4%).
CLHP ACE C18 CH₃CN (0.1%)/ H₂O (0.1% TFA) 70/30 Rt : 4.53 min
RMN 1H (DMSOd6) δ (ppm): 1,10-2,20 (19H, m); 3,40 (1H, q); 3,7 (1H, m); 4,25 (1H, q); 6,20 (m, 1H); 7,85 (1H, d); 8,4 (1H, dd).

### Exemple 22: Acide 2-[(1-Acetyl-4-{Hydroxy-[1-(1-isobutyryloxy-ethoxycarbonylamino)-ethyl]-phosphinoylmethyl}-piperidine-4-carbonyl)-amino]-propionique

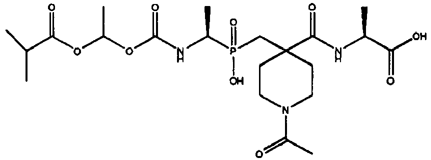

### Etape 1: Ester t-butylique de l'acide 1-Acetyl-piperidine-4-carboxylique

L'acide 1-acetylpiperidine-4-carboxylique (2,97g), mis en suspension dans 25 mL d'un mélange THF/toluène est chauffé à 85°C sous courant d'azote. Le N,N diméthylformamide dit-butyl acétal (25 mL, 6eq) est ajouté goutte à goutte et le chauffage est maintenu pendant 30 min. Le mélange est évaporé à sec et le résidu est repris par de l'acétate d'éthyle. La phase organique est lavée à l'eau, par une solution saturée de NaCl, séchée sur Na₂SO₄, filtrée et évaporée à sec. Huile jaune p=3,45g (Rdt 88%)
CLHP Atlantis T3 gradient 10-90 de CH₃CN (0.1%TFA/H₂O (0.1% TFA) en 15 min Rt=10,3 min.
ESI (+) (M+H)⁺=228
RMN (DMSO d6) δ (ppm): 1,20-1,80 (13H, m), 2,10 (3H, s), 2,40 (1H, m), 2,60-3,20 (4H, m), 3,60-4,30 (4H, m).

### Etape 2: Ester t-butylique de l'acide 1-Acetyl-4-hydroxymethyl-piperidine-4-carboxylique

A une solution à 0°C de iPr₂NH (2.10 mL, 14.89 mmol) dans le THF (16 mL) est additionné du nBuLi dans l'hexane (2.5 M) (6,8mL, 16,92 mmol, 2,5 eq). Après 30 min, le mélange est refroidi à -78°C et une solution du composé de l'étape 1 (1,54g 6,77 mmol) dans 9 mL de THF est ajoutée sous azote en 30 min. Le mélange est agité 1h à -78°C puis le paraformaldéhyde (5éq, 1,01g) est ajouté et le mélange réactionnel est ramené à température ambiante sous agitation. Après 30 min à TA, le mélange est partagé entre une solution saturée de NH₄Cl (120 mL) et AcOEt (60 mL).La phase aqueuse est extraite par AcOEt (2x40mL). La phase organique est lavée par HCl 1N, NaCl sat., séchée sur Na₂SO₄ et concentrée sous pression réduite pour donner une huile jaune P=1,20g.

Le produit brut est purifié par CLHP semi-préparative sur colonne Atlantis 30x100mm, éluant CH₃CN (0.01% TFA)/H₂O (0.01% TFA) 15/85. Rdt : 27%. CLHP Atlantis T3 20 % pendant 10 min puis gradient 20-90 de CH₃CN (0.1%TFA/H₂O (0.1% TFA) en 15 min Rt= 8,85 min.
ESI (+) (M+H)⁺=258,2
RMN (DMSO d6) δ (ppm): 1,20-1,80 (13H, m), 2,10 (3H, s), 2,60-3,20 (4H, m), 3,40 (2H, s), 3,60-4,30 (4H, m).

### Etape 3: Ester tert-butylique de l'acide 1-Acetyl-4-trifluoromethanesulfonyloxymethyl-piperidine-4-carboxylique

Un mélange de pyridine de 2.0 mL (250mmol) et de 20 mL de dichlorométhane est refroidi à -78°C sous azote. L'anhydride triflique (2.10 mL, 12.5mmol) est ajouté goutte à goutte. Après 10 min, une solution de 1.29g du composé de l'étape 2 (5.0mmol) dans 15mL de CH₂Cl₂ est ajoutée goutte à goutte. Le mélange est agité pendant 2h à -78°C. La phase organique est lavée par HCl 1N, H₂O, NaCl sat., séchée sur Na₂SO₄ et concentrée sous pression réduite pour donner 1.68g du produit attendu (Rdt : 86%) utilisé tel quel pour l'étape suivante.
CLHP Atlantis T3 (4.6*100mm, 3µm) 20 % pendant 10 min puis gradient 20-90% de CH₃CN (0.1%TFA/H₂O (0.1% TFA) en 15 min Rt= 22,7 min.
ESI (+) (M+H)⁺=390,2
RMN (CDCl₃) δ (ppm): 1,40-1,60 (11H, m), 1,90-2,30 (5H, m), 2,90 (2H, m), 3,40-3,80 (2H,m), 4,30-4,60 (2H, m).

### Etape 4 : Acide 1-acetyl-4-((((R)-1-(benzyloxycarbonylamino)ethyl)(hydroxy)phosphoryl) methyl)piperidine-4-carboxylique

Une solution d' iPr₂NH (0.61 mL, 4.31 mmol) dans THF (5mL) est refroidie à 0°C puis 3.1 mL de nBuLi 1.5 M dans l'hexane (4.67 mmol, 3.0eq) sont ajoutés. Après 15 min, le mélange est refroidi à -78°C et une solution du composé de l'étape 3 (925mg, 3.59 mmol) dans 8 mL de THF est ajouté sous azote de manière à maintenir la température inférieure à -60°C. Le mélange est agité 10 min à -78°C puis le composé de l'étape 4 (4.31 mmol) est ajouté dans 10 mL de THF et le mélange est agité pendant 10 min à -78°C puis pendant 5h à température ambiante.

Le mélange est dilué par de l'AcOEt. La phase organique est lavée par HCl 1N, NaHCO₃ 10%, NaCl sat., séchée sur Na₂SO₄ et concentrée sous pression réduite.

Le produit brut obtenu est mis en solution dans 31 mL de CH₂Cl₂ et 12 mL de TFA sont ajoutés. Le mélange est agité 2h à température ambiante. Après évaporation à sec, le produit attendu est purifié par CLHP semipréparative sur colonne Atlantis T3, CH₃CN (0.1% TFA)/ H₂0 (0.1 % TFA) 15/85 pour donner 367 mg du produit pur (Rdt : 23%).
RMN (DMSO d6) δ (ppm): 1.17 (3H, m), 1.40-1.95 (9H, m), 3.00 (1H, m), 3.23 (1H, m), 3.55-3.81 (2H, m), 5.03 (dd, 2H), 7.33 (5H, m), 7.46 (1H, d)
CLHP Atlantis T3 4.6x100mm, 3µm, CH₃CN (0.1% TFA)/H₂O (0.1% TFA) 20/80, Rt : 7.67 min.

### Etape 5: Trifluoroacétate de l'acide (2S)-2-(1-acetyl-4-((((R)-1-aminoethyl)(hydroxy)phosphoryl)methyl)piperidine-4-carboxamido)propanoique

Le composé de l'étape 4 (0,175 g, 0.41 mmol) est solubilisé dans 2 mL de DMF sous azote, puis on ajoute successivement le chlorhydrate de l'alaninate de t-butyle (89 mg, 1.2eq), 360µL de DIEA (5 eq) et 395 mg de TBTU (3eq). Le mélange est agité 15 min à température ambiante, puis le mélange réactionnel est évaporé à sec. Le résidu brut obtenu est solubilisé dans un mélange HBr/AcOH (4 mL) et la solution est agitée 1h à température ambiante. Le mélange est ensuite évaporé sous pression réduite. Le produit est purifié par CLHP semi-préparative sur colonne Atlantis T3 avec un gradient de 0 à 30% de CH₃CN en 30 min d'un mélange CH₃CN (0.1%TFA)/ H₂O (0.1% TFA) pour donner 80.8 mg du produit attendu (Rdt : 40.9%). CLHP Atlantis T3 avec un gradient de 0 à 30% de CH₃CN en 30 min Rt : 8.59 min RMN 1H (DMSOd6) δ (ppm): 1,15-1,35 (9H, m), 1,50-2,20 (10H, m), 3.23 (1H, m), 3,40 (1H, q), 4,25 (1H, q), 7,85 (1H, d), 8,10 (3H , m).

### Etape 6: Acide 2-[(1-Acetyl-4-{Hydroxy-[1-(1-isobutyryloxy-ethoxycarbonylamino)-ethyl]-phosphinoylmethyl}-piperidine-4-carbonyl)-amino]-propionique

80 mg (0.166 mmol) du composé de l'étape 5 est solubilisé dans 2 mL de CH₃CN et 560 µL de NaHCO₃ 2N. 52 mg de isobutyloxy succinimide carbonate (1.2 éq.) sont ajoutés le mélange est agité 1 h à 60°C. Le mélange est ensuite repris par de l'acétate d'éthyle, lavé par H₂O et par une solution aqueuse saturée de NaCl, séché sur Na₂SO₄ et évaporé à sec. Le produit brut est purifié par CLHP pour donner 70 mg du produit attendu (81.4%).
CLHP ACE C18 CH₃CN (0.1%)/ H₂O (0.1% TFA) 50/50 Rt : 5.37 min
RMN 1H (DMSOd6) δ (ppm): 1,10-2,20 (29H, m), 3.20 (1H, m), 3,40 (1H, q), 3,7 (1H, m), 4,25 (1H, q), 6,20 (m, 1H), 7,85 (1H, d), 8,10 (3H, m).

### Résumé des molécules décrites

Cette liste n'est pas limitative

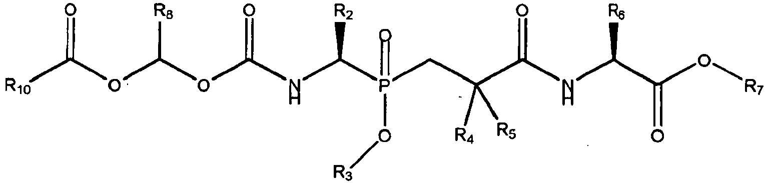

| Exemple | R₁₀ | R₈ | R₃ | R₂ | R₄ | R₅ | R₆ | R₇ |
|---|---|---|---|---|---|---|---|---|
| 3 | iPr | CH₃ | H | (R)-CH₃ | (S)-4-Phényl-Benzyl | H | CH₃ | CH₂Ph |
| 4 | iPr | CH₃ | H | (R)-CH₃ | (S)-4-Phényl-Benzyl | H | CH₃ | H |
| 6 | iPr | CH₃ | H | (R)-CH₃ | (S)-4-Phényl-Benzyl | H | CH₃ | C₂H₅ |
| 8 | iPr | CH₃ | H | (R)-CH₃ | (S)-4-Phényl-Benzyl | H | CH₃ | CH(CH₃)OCOOEt |
| 9 | iPr | CH₃ | CH(CH₃)OCOiPr | (R)-CH₃ | (S)-4-Phényl-Benzyl | H | CH₃ | H |
| 11 | iPr | CH₃ | H | (R)-CH₃ | (S)-4-Bromo-Benzyl | H | CH₃ | CH₂Ph |
| 12 | iPr | CH₃ | H | (R)-CH₃ | (S)-4-Bromo-Benzyl | H | CH₃ | H |
| 16 | iPr | CH₃ | H | (R)-Phényl | (S)-4-(3-Thiophen)-Benzyl | H | CH₃ | H |
| 17 | iPr | CH₃ | H | (R)-Phényl | (S)-4-(3-Thiophen)-Benzyl | H | CH₂OH | H |
| 18 | iPr | CH₃ | H | (R)-3-Thiophen | (S)-4-Phényl-Benzyl | H | CH₃ | H |
| 19 | iPr | H | H | (R)-CH₃ | (S)-4-Phényl-Benzyl | H | CH₃ | H |
| 20 | tBu | CH₃ | H | (R)-CH₃ | (S)-4-Phényl-Benzyl | H | CH₃ | H |
| 21 | iPr | CH₃ | H | (R)-CH₃ | Cyclopentane | | CH₃ | H |
| 22 | iPr | CH₃ | H | (R)-CH₃ | 4(1-acetyl)piperidine | | CH₃ | H |

### Exemple 23 : Etude de la stabilité de dérivés phosphiniques protégés par un groupe acyloxyalkyle sur la fonction phosphinique et dont la fonction amine est libre

L'inhibition de la néprilysine (NEP) et de l'aminopeptidase N (APN) qui est requise pour protéger complètement les enképhalines de leur catabolisme exige la reconnaissance des sites actifs des deux métallopeptidases à zinc par la même molécule dans la présente invention. Ceci a été obtenu, dans l'art antérieur, avec des dérivés phosphiniques décrits notamment dans la demande de brevet FR 2 755 135. Toutefois, pour des raisons de biodisponibilité par voie parentérale, il a été nécessaire de protéger de manière temporaire la fonction phosphinique par un groupe acyloxyalkyle (prodrogue) et dans certains cas également la fonction carboxyle des inhibiteurs décrits dans l'art antérieur.

Il est essentiel par ailleurs de noter que la reconnaissance de l'APN et son inhibition exige impérativement la présence d'une fonction amine libre.

Or, l'étude en solution des prodrogues décrites dans l'art antérieur montre que la protection du groupe phosphinique par un reste acyloxyalkyle conduit à un transfert d'une partie de ce groupe vers la fonction amine pour donner naissance à une molécule possédant une fonction amide à la place de l'amine libre nécessaire à l'affinité pour l'APN.

De ce fait, le composé formé, non hydrolysable, est totalement inactif vis à vis de cette enzyme. Ces composés ne peuvent donc être utilisés du fait de leur instabilité dans des préparations utilisées en clinique humaine.

L'étude en solution d'un composé de l'art antérieur a été réalisée comme suit :

Le composé de l'art antérieur est mis en solution dans différents mélanges utilisés pour une administration parentérale, la solution étant ensuite suivie dans le temps par CLHP (Colonne Kromasil C18 4,5*250mm, 50%CH₃CN(0,1 %TFA)/50% H₂O (0,1% TFA)) pour déterminer la composition de la solution. Dans tous les cas, on observe la formation au cours du temps de deux produits, l'acide phosphonique libre (composé actif) et le produit de transfert comme illustré sur le schéma suivant :

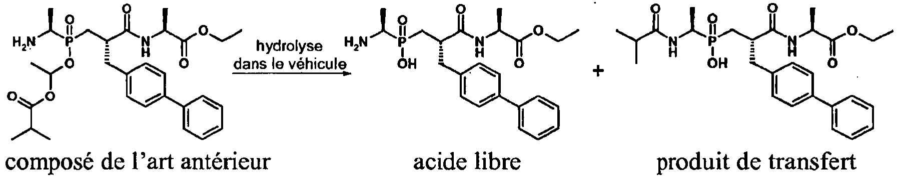

Ainsi, dans le cas d'une solution du composé de l'art antérieur dans un mélange éthanol/cremophor/H₂O (1/1/8), la teneur en composé de départ dans le véhicule diminue au cours du temps, comme le montre le tableau 1 suivant :

| Temps après solubilisation | 0 h | 3 h | 6 h | 96 h |
|---|---|---|---|---|
| % composé de départ | 90,5% | 82,0% | 75,7% | 25,1% |

### Exemple 25 : Résultats pharmacologiques

Les molécules de la présente invention ont été étudiées pour leur action analgésique sur les modèles animaux les plus prédictifs de la réponse chez l'homme. Les tests privilégiés sont ceux qui s'adressent aux douleurs neuroinflammatoires (NI) et neuropathiques (NP) chez le rat et la souris.

Les molécules de la présente invention se sont révélées actives sur les tests suivants chez la souris :
i) douleur provoquée par l'administration de formaline dans la patte et étude de la réponse analgésique dans la première phase considérée comme reflétant une action périphérique au niveau des nocicepteurs, et
ii) hyperalgésie et allodynie produites par compression partielle et unilatérale du nerf sciatique (modèle de Seltzer) (Bennett G.J. et Xie Y.K., Pain (1998) 33, 87-107).

Les techniques utilisées dans ces tests sont décrites en détail et répertoriées dans des revues telles que : M.J. Millan. The induction of pain: an integrative review, in Progress in Neurobiology (1999), 52, 1-164.

L'association et la synergie entre un opioîde et la gabapentine a été rapportée, en particulier dans la reference: Menendez et al (2008), Eur. J. Pharmacol, 596,50-55. Les tests suivants ont ainsi été réalisés.

### A/ Test à la formaline (phase I)

Les molécules ont été étudiées à un temps (90 mn) pour des essais comparatifs et à deux temps, 90 et 150 minutes, de manière à observer leur durée d'action.

### Description du test :

Les animaux (souris mâle OF1) proviennent de l'élevage Charles River (France) et pèsent 25-35 g au début de l'expérience. Le poids de chaque souris est pris en compte pour l'administration du produit.

Le test est basé sur le protocole décrit par S. HUNSKAAR et al., Formalin test in mice, a useful technique for evaluating mild analgesics, J. Neurosci. Methods (1995), 14, 69-75. C'est la phase précoce du test (5 à 10 min après injection de formaline), qui est considérée comme reflétant une douleur de type neuropathique, qui est étudiée.

Les souris (n=8) sont placées individuellement dans une enceinte transparente (50x25 cm²) et sont habituées à cet environnement pendant 20 minutes. Après cette période, 20 µl de formaline (5% HCHO) en solution dans du sérum physiologique (H₂O, NaCl 0,9%), sont injectés par voie sous-cutanée sur la face plantaire de la patte droite de l'animal. On utilise une seringue de 26 connectée à une micro-seringue. Chaque souris est ensuite immédiatement replacée dans l'enceinte du test et des réponses douloureuses (nociceptives) sont mesurées durant 5 minutes (phase précoce). Seuls les lèchements de la patte sont comptés.

L'activité analgésique est testée après gavage des animaux à différents temps (généralement 20 mn, 90 mn et 150 mn) après l'injection de formaline, par :
- le véhicule seul (éthanol, méthylcellulose 0,5% dans l'eau)
- le véhicule et un composé de l'invention (50 mg/kg)

L'action analgésique du produit est mesurée par la diminution du nombre de lèchements de la patte lésée, par rapport au nombre de lèchements de l'animal qui a reçu le véhicule seul. La durée totale (discontinue) des léchements, exprimée en secondes pour chaque souris, est comptée durant 4 min. Les valeurs cumulées pour n souris sont ensuite divisées par le nombre n de souris étudiées.

Les résultats sont indiqués pour les trois composés des exemples 3, 4 et 8 sur la **figure 1****.**

Les trois composés montrent des effets analgésiques puissants (40 à 60%) caractérisés par un abaissement très significatif du nombre de lèchements par rapport au véhicule (contrôle) et les effets sont à peu près constants durant la période du test. Ainsi, on observe une activité analgésique des composés de l'invention à des temps allant jusqu'à 150 mn ce qui atteste de la longue durée d'action des composés de l'invention.

L'action analgésique est bloquée par une pré-administration d'un antagoniste, le méthyl-naloxonium, qui, à la dose utilisée (2 mg/kg), est incapable de franchir la barrière hématoméningée (Milne R.J. et al., Neurosci. Lett. (1990), 114, 25-32), démontrant que l'activité de ces molécules s'exerce au niveau périphérique (nocicepteurs) où ils augmentent les enképhalines libérées au site lésé. Ces résultats montrent bien que les composés selon l'invention ne franchissent pas la barrière hématoméningée.

### B/ Etude comparative de l'effet analgésique du composé de l'exemple 8 et d'une molécule de référence de l'art antérieur

La molécule de référence de l'art antérieur utilisée pour cette étude a la structure suivante :

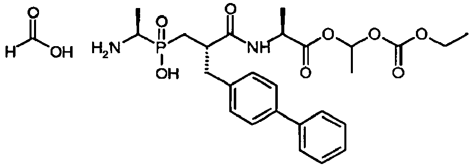

Le test A décrit précédemment a été réitéré avec le composé de l'exemple 8 (50 mg/kg ; figure 2A) et la molécule de référence de l'art antérieur (100 mg/kg ; figure 2B) afin de comparer leur action dans le temps, à 90 mn après l'injection de formaline.

On constate ainsi que la molécule de référence ne possède aucune activité à 90 minutes à une dose de 100 mg/kg (per os) alors que le composé de l'exemple 8 conduit au contraire à une activité analgésique très significative à 90 minutes à une dose de 50 mg/kg (per os) deux fois inférieure (voir figure 2).

La présente invention se caractérise donc par la mise au point de molécules possédant des propriétés analgésiques par voie orale avec une longue durée d'action.

### C/ Effets antiallodynique et antihyperalgésique du composé de l'exemple 3 après administration orale chez la souris.

Ce test a été décrit en détail par A.B. Malmberg et A.I. Basbaum, Partial Sciatic nerve injury in the mouse as a model of neuropathic pain: behavioural and neuroanatomical correlates. Pain, (1998) 76, 215-222.

Il a été réalisé sur des souris mâles OF1 (Charles River, n=39), de 18 à 20 g, par ligature partielle du nerf sciatique du côté ipsilatéral. Les animaux sont testés dans une période de 3 à 26 jours après l'opération.

La mesure de l'hyperalgésie a été effectuée selon la méthode décrite par K. Hargreaves et al., A new sensitive method for measuring thermal nociception in cutaneous hyperalgesia, Pain, (1988), 32, 77-88, en utilisant comme source de chaleur, l'appareil « Plantar test » (Bioseb, France). L'intensité du stimulus nociceptif est calibrée à 8-10 s avec un seuil d'arrêt automatique (cut-off time) à 20 secondes. La moyenne des retraits de la patte induite par la chaleur, a été mesurée sur les pattes ipsilatérales (nerf endommagé) et contralatérales (nerf intact). Chaque mesure est effectuée 3 fois sur chaque patte.

L'allodynie mécanique est mesurée comme décrit par S.R. Chaplan et al., Quantitative assessement of tactile allodynia in the rat paw, J. Neurosci. Meth. (1994), 53, 55-63. Les pattes ipsilatérale (lésion) et contralatérale (contrôle) sont testées comme précédemment, l'effet antiallodynique mécanique étant mesuré par la méthode de Von Frey **(**Malmerg A.B. and Basbaum A.I.,1998, Pain, 76, 215-222**)** avec des filaments de taille croissante exerçant une pression également croissante.

### Effet antihyperalgésique :

Les résultats obtenus, représentés sur la **figure 3****,** montrent que, administré per os à 50 mg/kg, le composé de l'exemple 3 produit une diminution significative très importante (65-100%) de l'hyperalgésie thermique induite par la ligature partielle du nerf sciatique dans la période 45-120 mn avec un effet maximum à 90 mn.

### Effet Anti-allodynique :

L'effet du composé de l'exemple 3 sur l'allodynie mécanique est mesuré par le test de Von Frey. Les résultats obtenus, représentés sur la **figure 4****,** montrent un effet anti-allodynique significatif de longue durée (45-120 mn) avec un maximum à 60 mn correspondant à 75% de la réponse maximale (contrôle non traité).

### D/ Potentialisation des effets anti-allodyniques du composé de l'exemple 4 par association avec la gabapentine.

L'effet de l'association du composé de l'exemple 4 et de la gabapentine, les deux produits étant administrés par voie orale, est mesuré par le test de Von Frey. Les résultats obtenus, après 60 min, représentés sur la figure 5, montrent une très forte potentialisation de l'association (>300%) par rapport au composé de l'exemple 4 ou à la gabapentine utilisés seuls, qui se montrent inactifs aux mêmes doses. Aucun effet n'est obtenu sur la patte contralatérale non lésée

## Revendications

1. Composé de formule générale (I) suivante :
R₁-NH-CH(R₂)-P(=PO)(OR₃)-CH₂-C(R₄)(R₅)-CONH-CH(R₆)-COOR₇ (I)
ou un sel pharmaceutiquement acceptable de celui-ci,
pour laquelle:
▪ R₁ représente un groupement -C(=O)-O-C(R⁸)(R⁹)-OC(=O)-R¹⁰, dans lequel
∘ R⁸ et R⁹ représentent, indépendamment l'un de l'autre, un atome d'hydrogène ou un groupe alkyle,
∘ et R¹⁰ représente un groupe alkyle,
▪ R₂ représente :
- un groupe alkyle linéaire ou ramifié, comportant de 1 à 6 atomes de carbone
- un groupe phényle
▪ R₃ représente un atome d'hydrogène ou un groupe de formule -C(R¹²)(R¹³)-OC(=O)-R¹⁴, dans lequel
∘ R¹² et R¹³ représentent, indépendamment l'un de l'autre, un atome d'hydrogène ou un groupe alkyle,
∘ et R¹⁴ représente un groupe alkyle ;
▪ R₅ représente un atome d'hydrogène et R₄ représente :
- un benzyle éventuellement substitué sur le noyau phényle par :
∘ 1 à 5 atome(s) d'halogène tel(s) que le fluor ou le brome ;
∘ un groupe OR¹⁵ ou SR¹⁵, dans lequel R¹⁵ représente un atome d'hydrogène, un groupe benzyle ou une chaîne hydrocarbonée, saturée ou insaturée, linéaire ou ramifiée, comportant de 1 à 4 atomes de carbone ;
∘ un groupe amino ;
∘ un groupe CF₃ ;
∘ un groupe phényle ; ou
∘ un cycle hétéroaromatique à 5 ou 6 chaînons ;
R₄ et R₅ forment ensemble, avec le carbone qui les porte :
- un cycle hydrocarboné saturé à 5 ou 6 chaînons ; ou
- un cycle pipéridine, l'azote se trouvant en position 4 et étant éventuellement substitué par :
* un groupe -SO₂-Ph ;
* un groupe CF₃ ;
* un groupe alkyle en C₁ à C₄ ;
* un groupe acyle en C₁ à C₄ ;
* un phényle ou un benzyle éventuellement substitué par un ou plusieurs atome(s) d'halogène, un groupe alkyle en C₁ à C₄ ou un groupe alcoxy en C₁ à C₄ ; ou
* un hétérocycle aromatique tel qu'une pyridine ou une pyrimidine, éventuellement substitué par un groupe alkyle en C₁ à C₄ ou un groupe alcoxy en C₁ à C₄ ;
▪ R₆ représente :
- un groupe alkyle, linéaire ou ramifié, comportant de 1 à 6 atomes de carbone, éventuellement substitué par :
∘ un groupe OR¹⁶, dans lequel R¹⁶ représente un atome d'hydrogène,
▪ R₇ représente un radical choisi dans le groupe constitué par un atome d'hydrogène, un benzyle, un alkyle en C₂ à C₄, -CHR¹⁸-COOR¹⁹, -CHR¹⁸-OC(=O)R¹⁹ et -CHR¹⁸-OC(=O)OR¹⁹, dans lesquels R¹⁸ et R¹⁹ représentent, indépendamment l'un de l'autre, un groupe alkyle, aryle, arylalkyle, cycloalkyle, cyclohétéroalkyle, hétéroalkyle, hétéroaryle ou hétéroarylalkyle.

2. Composé selon la revendication 1, **caractérisé en ce que** R₁ représente un groupe -(C=O)O-CHMe-OC(=O)CHMe₂.

3. Composé selon l'une quelconque des revendications 1 ou 2, **caractérisé en ce que** R₃ représente un atome d'hydrogène ou un groupe -CHMe-OC(=O)CHMe₂, et de préférence représente un atome d'hydrogène.

4. Composé selon l'une quelconque des revendications 1 à 3, **caractérisé en ce que** R₆ représente un groupe alkyle tel qu'un groupe méthyle.

5. Composé selon l'une quelconque des revendications 1 à 4, **caractérisé en ce que** R₇ représente un atome d'hydrogène ou un groupe alkyle, tel qu'un éthyle, ou un groupe benzyle ou un groupe -CH(CH₃)-O-C(=O)-O-Et.

6. Composé ou sel pharmaceutiquement acceptable de celui-ci, choisi parmi les composés suivants :
Ester benzylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3- {hydroxy-[1-(1-isobutyryloxy-éthoxycarbonyl amino)-éthyl]-phosphinoyl} -propionylamino)-propionique
Ester éthylique de l'acide 2-(2-Biphényl-4-ylméthyl-3-{hydroxy-[1-(1-isobutyryloxy-éthoxycarbonylamino)-éthyl]-phosphinoyl}-propionylamino)-propionique Ester éthoxycarbonyloxy de l'acide 2-(2-Biphényl-4-ylméthyl-3-{hydroxy-[1-(1-isobutyryloxy-éthoxycarbonylamino)-éthyl]-phosphinoyl}-propionylamino)-propionique
Acide 2-(2-Biphényl-4-ylméthyl-3- {(1-isobutyryloxy-éthoxy)-[1-(1-isobutyryloxy-éthoxycarbonylamino)-éthyl]-phosphinoyl}-propionylamino)-propionique
Ester benzylique de l'acide 2-(2-(4-Bromo-benzyl)-3-{hydroxy-[1-(1-isobutyryloxy-éthoxycarbonylamino)-éthyl]-phosphinoyl}-propionylamino)-propionique
Acide 2-(2-(4-Bromo-benzyl)-3-{hydroxy-[1-(1-isobutyryloxy-éthoxycarbonylamino)-éthyl]-phosphinoyl}-propionylamino)-propionique
Acide 2-[2-{Hydroxy-[(1-isobutyryloxy-ethoxycarbonylamino)-phenyl-methyl]-phosphinoylmethyl}-3-(4-thiophen-3-yl-phenyl)-propionylamino]-propionique
Acide 2-[2- {Hydroxy-[(1-isobutyryloxy-ethoxycarbonylamino)-phenyl-methyl]-phosphinoylmethyl}-3-(4-thiophen-3-yl-phenyl)-propionylamino]-3-hydroxypropionique
Acide 2-(3-Biphenyl-4-yl-2-{hydroxy-[(1-isobutyryloxy-ethoxycarbonylamino)-thiophen-3-yl-methyl]-phosphinoylmethyl}-propionylamino)-propionique
Acide 2- {3-Biphenyl-4-yl-2-[hydroxy-(1-isobutyryloxymethoxy carbonylamino-ethyl)-phosphinoylmethyl]-propionylamino}-propionique
Acide 1-(1-{[3-biphenyl-4-yl-2-(1-carboxy-ethylcarbamoyl)-propyl]-hydroxy-phosphinoyl}-ethylcarbamoyloxy)-ethyl de l'acide 2-Dimethyl-propionique
Acide 2-[(1-{Hydroxy-[1-(1-isobutyryloxy-ethoxycarbonylamino)-ethyl]-phosphinoylmethyl}-cyclopentanecarbonyl)-amino]-propionique
Acide 2-[(1-Acétyl-4-{hydroxy-[1-(1-isobutyryloxy-éthoxycarbonylamino)-éthyl]-phosphinoylméthyl}-pipéridine-4-carbonyl)-amino]-propionique.

7. Composé selon l'une quelconque des revendications 1 à 6 pour son utilisation en tant que médicament, notamment destiné au traitement de la douleur telle que des douleurs vives ou des douleurs neuropathiques ou neuroinflammatoires.

8. Composition pharmaceutique comprenant au moins un composé de formule (I) selon l'une quelconque des revendications 1 à 6 et au moins un véhicule pharmaceutiquement acceptable.

9. Composition pharmaceutique selon la revendication 8, **caractérisée en ce que** le véhicule est approprié pour une administration par voie orale.

10. Composition pharmaceutique selon l'une quelconque des revendications 8 et 9, pour son utilisation en tant que médicament destiné au traitement des douleurs neuropathiques ou inflammatoires, causées notamment par un diabète sucré de type I ou II, une infection virale ou rétrovirale, une chimiothérapie d'un cancer, une radiothérapie, une intervention chirurgicale incluant l'illusion des amputés et les séquelles d'une mastectomie, l'alcoolisme, une névralgie faciale, un traumatisme tel qu'un syndrome du plexus brachial, une radiculopathie ou une radiculalgie telle qu'une sciatique, une cruralgie ou un syndrome du défilé thoraco-brachial, une fibromyalgie, un syndrome des jambes sans repos, une douleur articulaire inflammatoire causée notamment par l'arthrite ou une phase aigue de l'arthrose, une douleur articulaire dégénérative causée notamment par l'arthrose, ou encore une lombalgie.

11. Composition pharmaceutique selon la revendication 8, **caractérisée en ce que** le véhicule est approprié pour une administration par voie sublinguale, parentérale, sous-cutanée, pulmonaire, nasale, intramusculaire, intraveineuse, intrathécale, intra-articulaire ou transdermique.

12. Composition pharmaceutique selon la revendication 11, pour son utilisation en tant que médicament destiné au traitement des douleurs vives.

## Patentansprüche

1. Verbindung der folgenden allgemeinen Formel (I):
R₁-NH-CH(R₂)-P(=O)(OR₃)-CH₂-C(R₄)(R₅)-CONH-CH(R₆)-COOR₇ (I)
oder ein pharmazeutisch verträgliches Salz davon,
wobei:
▪ R₁ einen Rest -C(=O)-O-C(R⁸)(R⁹)-OC(=O)-R¹⁰ bedeutet, wobei
∘ R⁸ und R⁹ unabhängig voneinander ein Wasserstoffatom oder einen Alkylrest bedeuten,
∘ und R¹⁰ einen Alkylrest bedeutet,
▪ R₂:
- einen Alkylrest, geradkettig oder verzweigt, mit 1 bis 6 Kohlenstoffatomen,
- einen Phenylrest
bedeutet,
▪ R₃ ein Wasserstoffatom oder einen Rest der Formel -C(R¹²)(R¹³)-OC(=O)-R¹⁴ bedeutet, wobei
∘ R¹² und R¹³ unabhängig voneinander ein Wasserstoffatom oder einen Alkylrest bedeuten,
∘ und R¹⁴ einen Alkylrest bedeutet;
▪ R₅ ein Wasserstoffatom bedeutet und R₄:
- ein Benzyl bedeutet, das am Phenylring gegebenenfalls substituiert ist mit:
∘ 1 bis 5 Halogenatom(en), wie Fluor oder Brom,
∘ einem Rest OR¹⁵ oder SR¹⁵, wobei R¹⁵ ein Wasserstoffatom, einen Benzylrest oder eine gesättigte oder ungesättigte, geradkettige oder verzweigte Kohlenwasserstoffkette mit 1 bis 4 Kohlenstoffatomen bedeutet;
∘ einer Aminogruppe;
∘ einem Rest CF₃;
∘ einem Phenylrest; oder
∘ einem heteroaromatischen 5- oder 6-gliedrigen Ring;
R₄ und R₅ zusammen mit dem Kohlenstoff, der sie trägt:
- einen gesättigten 5- oder 6-gliedrigen Kohlenwasserstoffring; oder
- einen Piperidinring bilden, wobei sich der Stickstoff an Position 4 befindet und gegebenenfalls substituiert ist mit:
* einem Rest -SO₂-Ph;
* einem Rest CF₃;
* einem C₁-C₄-Alkylrest;
* einem C₁-C₄-Acylrest;
* einem Phenyl oder einem Benzyl, gegebenenfalls substituiert mit einem oder mehreren Halogenatom(en), einem C₁-C₄-Alkylrest oder einem C₁-C₄-Alkoxyrest; oder
* einem aromatischen Heterocyclus, wie einem Pyridin oder einem Pyrimidin, gegebenenfalls substituiert mit einem C₁-C₄-Alkylrest oder einem C₁-C₄-Alkoxyrest;
▪ R₆:
- einen geradkettigen oder verzweigten Alkylrest mit 1 bis 6 Kohlenstoffatomen bedeutet, der gegebenenfalls substituiert ist mit:
o einem Rest OR¹⁶; wobei R¹⁶ ein Wasserstoffatom bedeutet;
▪ R₇ einen Rest bedeutet, der ausgewählt ist aus der Gruppe bestehend aus einem Wasserstoffatom, einem Benzyl, einem C₂-C₄-Alkyl, -CHR¹⁸-COOR¹⁹, -CHR¹⁸-OC(=O)R¹⁹ und -CHR¹⁸-OC(=O)OR¹⁹, wobei R¹⁸ und R¹⁹ unabhängig voneinander einen Alkyl-, Aryl-, Arylalkyl-, Cycloalkyl-, Cycloheteroalkyl-, Heteroalkyl-, Heteroaryl- oder Heteroarylalkylrest bedeuten.

2. Verbindung nach Anspruch 1, **dadurch gekennzeichnet, dass** R₁ einen Rest -(C=O)O-CHMe-OC(=O)CHMe₂ bedeutet.

3. Verbindung nach einem der Ansprüche 1 oder 2, **dadurch gekennzeichnet, dass** R₃ ein Wasserstoffatom oder einen Rest -CHMe-OC(=O)CHMe₂ bedeutet und vorzugsweise ein Wasserstoffatom bedeutet.

4. Verbindung nach einem der Ansprüche 1 bis 3, **dadurch gekennzeichnet, dass** R₆ einen Alkylrest, wie z.B. eine Methylgruppe, bedeutet.

5. Verbindung nach einem der Ansprüche 1 bis 4, **dadurch gekennzeichnet, dass** R₇ ein Wasserstoffatom oder einen Alkylrest, wie z.B. ein Ethyl, oder einen Benzylrest oder einen Rest -CH(CH₃)-O-C(=O)-O-Et bedeutet.

6. Verbindung oder pharmazeutisch verträgliches Salz davon, ausgewählt aus den folgenden Verbindungen:
2-((2-Biphenyl-4-ylmethyl-3-{hydroxy-[1-(1-isobutyryloxy-ethoxycarbonylamino)-ethyl]-phosphinoyl}-propionylamino)-propionsäurebenzylester
2-((2-Biphenyl-4-ylmethyl-3-{hydroxy-[1-(1-isobutyryloxy-ethoxycarbonylamino)-ethyl]-phosphinoyl} -propionylamino)-propionsäure
2-((2-Biphenyl-4-ylmethyl-3-{hydroxy-[1-(1-isobutyryloxy-ethoxycarbonylamino)-ethyl]-phosphinoyl}-propionylamino)-propionsäureethylester
2-((2-Biphenyl-4-ylmethyl-3-{hydroxy-[1-(1-isobutyryloxy-ethoxycarbonylamino)-ethyl]-phosphinoyl}-propionylamino)-propionsäureethoxycarbonyloxyester
2-((2-Biphenyl-4-ylmethyl-3-{(1-isobutyryloxy-ethoxy)-[1-(1-isobutyryloxy-ethoxycarbonylamino)-ethyl]-phosphinoyl}-propionylamino)-propionsäure 2-(2-(4-Brombenzyl)-3-{hydroxy-[1-(1-isobutyryloxy-ethoxycarbonylamino)-ethyl]-phosphinoyl}-propionylamino)-propionsäurebenzylester
2-((2-(4-Brombenzyl)-3-{hydroxy-[1-(1-isobutyryloxy-ethoxycarbonylamino)-ethyl]-phosphinoyl} -propionylamino)-propionsäure
2-[2-{Hydroxy-[(1-isobutyryloxy-ethoxycarbonylamino)-phenyl-methyl]-phosphinoylmethyl}-3-(4-thiophen-3-yl-phenyl)-propionylamino]-propionsäure
2-[2-{Hydroxy-[(1-isobutyryloxy-ethoxycarbonylamino)-phenyl-methyl]-phosphinoylmethyl}-3-(4-thiophen-3-yl-phenyl)-propionylamino]-3-hydroxypropionsäure
2-((3-Biphenyl-4-yl-2-{hydroxy-[(1-isobutyryloxy-ethoxycarbonylamino)-thiophen-3-yl-methyl] -phosphinoylmethyl} -propionylamino)-propionsäure
2-{3-Biphenyl-4-yl-2-[hydroxy-(1-isobutyryloxymethoxycarbonylamino-ethyl)-phosphinoylmethyl]-propionylamino}-propionsäure
2-Dimethylpropionsäure-1-(1-{[3-Biphenyl-4-yl-2-(1-carboxy-ethylcarbamoyl)-propyl]-hydroxy-phosphinoyl}-ethylcarbamoyloxy)-ethylsäure
2-[((1-{Hydroxy-[1-(1-isobutyryloxy-ethoxycarbonylamino)-ethyl]-phosphinoylmethyl}-cyclopentancarbonyl)-amino]-propionsäure
2-[((1-Acetyl-4-{hydroxy-[1-(1-isobutyryloxy-ethoxycarbonylamino)-ethyl]-phosphinoylmethyl}-piperidin-4-carbonyl)-amino]-propionsäure.

7. Verbindung nach einem der Ansprüche 1 bis 6 zur Verwendung als Medikament, insbesondere zur Behandlung von Schmerz, wie z.B. starken Schmerzen oder neuropathischen oder neuroinflammatorischen Schmerzen.

8. Arzneimittel, umfassend mindestens eine Verbindung der Formel (I) nach einem der Ansprüche 1 bis 6 und mindestens einen pharmazeutisch verträglichen Träger.

9. Arzneimittel nach Anspruch 8, **dadurch gekennzeichnet, dass** der Träger für eine orale Verabreichung geeignet ist.

10. Arzneimittel nach einem der Ansprüche 8 und 9 zur Verwendung als Medikament zur Behandlung von neuropathischen oder inflammatorischen Schmerzen, insbesondere verursacht durch Diabetes mellitus vom Typ I oder II, eine Virus- oder Retrovirusinfektion, eine Chemotherapie gegen Krebs, eine Strahlentherapie, einen chirurgischen Eingriff, einschließlich des Phantomschmerzes bei Amputierten und der Folgen einer Mastektomie, Alkoholismus, Gesichtsneuralgie, einem Trauma, wie z.B. einem Brachialplexussyndrom, einer Radikulopathie oder einer Radikulalgie, wie z.B. einem Ischias, einer Kruralgie oder einem Syndrom des thorakobrachialen Engpasses, einer Fibromyalgie, einem Syndrom der ruhelosen Beine, einem entzündlichen Gelenksschmerz, insbesondere verursacht durch Arthritis oder eine akute Phase von Arthrose, einem degenerativen Gelenksschmerz, insbesondere verursacht durch Arthrose, oder auch einer Lumbalgie.

11. Arzneimittel nach Anspruch 8, **dadurch gekennzeichnet, dass** der Träger für eine sublinguale, parenterale, subkutane, pulmonale, nasale, intramuskuläre, intravenöse, intrathekale, intraartikuläre oder transdermale Verabreichung geeignet ist.

12. Arzneimittel nach Ansprüch 11 zur Verwendung als Medikament zur Behandlung von starken Schmerzen.

## Claims

1. Compound having the following general formula (I):
R₁-NH-CH(R₂) -P (=O)(OR₃)-CH₂-C(R₄)(R₅) -CONH-CH (R₆)-COOR₇ (I)
or a pharmaceutically acceptable salt thereof,
where:
- R₁ is a group -C(=O)-O-C(R⁸)(R⁹)-OC(=O)-R¹⁰, wherein
--- R⁸ and R⁹ are, independently of each other, a hydrogen atom or an alkyl group,
--- and R¹⁰ is an alkyl group,
- R₂ is:
-- an alkyl group linear or branched, comprising 1 to 6 carbon atoms
-- a phenyl group
- R₃ is a hydrogen atom or a group having the formula
- C(R¹²)(R¹³)-OC(=O)-R¹⁴, wherein
--- R¹² and R¹³ are, independently of each other, a hydrogen atom or an alkyl group,
--- and R¹⁴ is an alkyl group;
- R₅ is a hydrogen atom and R₄ is:
-- a benzyl optionally substituted on the phenyl nucleus by:
--- 1 to 5 halogen atoms such as fluorine or bromine;
--- a group OR¹⁵ or SR¹⁵, wherein R¹⁵ is a hydrogen atom, a benzyl group or a linear or branched saturated or unsaturated hydrocarbon chain, comprising 1 to 4 carbon atoms;
--- an amino group;
--- a CF₃ group;
--- a phenyl group; or
--- a heteroaromatic 5- or 6-membered cycle ;
R₄ and R₅ form together, with the carbon bearing same:
-- a saturated hydrocarbon 5- or 6-membered cycle, or
-- a piperidine cycle, thenitrogen being situated in position 4 and being optionally substituted by:
--- a -SO₂-Ph group;
--- a CF₃ group;
--- a C₁ to C₄ alkyl group;
--- a C₁ to C₄ acyl group;
--- a phenyl or a benzyl optionally substituted by one or a plurality of halogen atoms, a C₁ to C₄ alkyl group or a C₁ to C₄ alkoxy group; or
--- an aromatic heterocycle such as a pyridine or a pyrimidine, optionally substituted by a C₁ to C₄ alkyl group or a C₁ to C₄ alkoxy group;
- R₆ is:
-- a linear or branched alkyl group, comprising 1 to 6 carbon atoms, optionally substituted by:
--- a group OR¹⁶, wherein R¹⁶ is a hydrogen atom,
- R₇ is a radical selected in the group consisting of a hydrogen atom, a benzyl, a C₂ to C₄ alkyl, -CHR¹⁸-COOR¹⁹, ⁻CHR¹⁸-OC(=O)R¹⁹ and -CHR¹⁸-OC(=O)OR¹⁹, wherein R¹⁸ and R¹⁹ are, independently from each other, an alkyl, aryl,
arylalkyl, cycloalkyl, cycloheteroalkyl, heteroalkyl, heteroaryl or heteroarylalkyl group.

2. Compound according to claim 1, **characterized in that** R₁ is the group -(C=O)O-CHMe-OC(=O)CHMe₂.

3. Compound according to any of claims 1 and 2, **characterized in that** R₃ is a hydrogen atom or a - CHMe-OC(=O)CHMe₂ group, and preferably is a hydrogen atom.

4. Compound according to any of claims 1 to 3, characterized that R₆ is an alkyl group such as a methyl group.

5. Compound according to any of claims 1 to 4, **characterized in that** R₇ is a hydrogen atom or an alkyl group, such as an ethyl, or a benzyl or a - CH(CH₃)-O-C(=O)-O-Et group.

6. Compound or pharmaceutically acceptable salt thereof, selected from the following compounds:
2-((2-Biphenyl-4-ylmethyl-3-{hydroxy-[1-(1-isobutyryl-oxy-ethoxycarbonylamino)-ethyl]-phosphinoyl}-propionylamino)-propionic acid benzyl ester
2-((2-Biphenyl-4-ylmethyl-3-{hydroxy-[1-(1-isobutyryl-oxy-ethoxycarbonylamino)-ethyl]-phosphinoyl}-propionylamino)-propionic acid
2-((2-Biphenyl-4-ylmethyl-3-{hydroxy-[1-(1-isobutyryl-oxy-ethoxycarbonylamino)-ethyl]-phosphinoyl}-propionylamino)-propionic acid ethyl ester
Ethoxycarbonyloxy 2-(2-Biphenyl-4-ylmethyl-3-{hydroxy-[1-(1-isobutyryloxy-ethoxycarbonylamino)-ethyl]-phosphinoyl}-propionylamino)-propionic acid ester
2-((2-Biphenyl-4-ylmethyl-3-{(1-isobutyryloxy-ethoxy-[1-(1-isobutyryloxy-ethoxycarbonylamino)-ethyl]-phosphinoyl}-propionylamino)-propionic acid
2-((2-(4-Bromo-benzyl)-3-{hydroxy-[1-(1-isobutyryloxy-ethoxycarbonylamino)-ethyl]-phosphinoyl}-propionyl-amino)-propionic acid benzyl ester
2-((2-(4-Bromo-benzyl)-3-{hydroxy-[1-(1-isobutyryloxy-ethoxycarbonylamino)-ethyl]-phosphinoyl}-propionyl-amino)-propionic acid
2-[2-Hydroxy-[(1-isobutyryloxy-ethoxycarbonylamino)-phenyl-methyl]-phosphinoylmethyl}-3-(4-thiophen-3-yl-phenyl)-propionylamino]-propionic acid
2-[2-Hydroxy-[(1-isobutyryloxy-ethoxycarbonylamino)-phenyl-methyl]-phosphinoylmethyl}-3-(4-thiophen-3-yl-phenyl)-propionylamino]-3-hydroxypropionic acid
2-((3-Biphenyl-4-yl-2-{hydroxy-[(1-isobutyryloxy-ethoxycarbonylamino)-thiophen-3-yl-methyl]-phosphinoylmethyl}-propionylamino)-propionic acid
2-{3-Biphenyl-4-yl-2-[hydroxy-[(1-isobutyryloxy-methoxy carbonylamino-ethyl)-phosphinoylmethyl]-propionyl-amino}-propionic acid
1-((1-{[3-biphenyl-4-yl-2-(1-carboxy-ethylcarbamoyl)-propyl]-hydroxy-phosphinoyl}-ethylcarbamoyloxy)-ethyl acid of 2-dimethyl-propionic acid
2-[((1-{Hydroxy-[1-(1-isobutyryloxy-ethoxycarbonylamino)-ethyl]-phosphinoylmethyl}-cyclopentanecarbonyl)-amino]-propionic acid
2-[((1-Acetyl-4-{hydroxy-[1-(1-isobutyryloxy-ethoxy carbonylamino)-ethyl]-phosphinoylmethyl}-piperidine-4-carbonoyl)-amino]-propionic acid.

7. Compound according to any of claims 1 to 6 for use thereof as a medicinal product, particularly for the treatment of pain such as sharp pain or neuropathic or neuroinflammatory pain.

8. Pharmaceutical composition comprising at least one compound having formula (I) according to any of claims 1 to 6 and at least one pharmaceutical acceptable vehicle.

9. Pharmaceutical composition according to claim 8, **characterized in that** the vehicle is suitable for oral administration.

10. Pharmaceutical composition according to any of claims 8 and 9, for use thereof as a medicinal product for the treatment of neuropathic or inflammatory pain caused by diabetes mellitus type I or II, viral or retroviral infection, cancer chemotherapy, radiotherapy, a surgical procedure including amputee illusion and the after-effects of mastectomy, alcoholism, facial neuralgia, trauma such as brachial plexus syndrome, radiculopathy or radiculalgia such as sciatica, cruralgia or thoracic outlet syndrome, fibromyalgia, restless leg syndrome, inflammatory joint pain caused particularly by arthritis or an acute phase of arthrosis, degenerative joint pain caused particularly by arthrosis, or lumbago.

11. Pharmaceutical composition according to claim 8, **characterized in that** the vehicle is suitable for administration by sublingual, parenteral, subcutaneous, pulmonary, nasal, intramuscular, intravenous, intrathecal, intra-articular or transdermal route.

12. Pharmaceutical composition according to claim 11, for use thereof as a medicinal product for the treatment of sharp pain.
